# EUROPEAN PATENT APPLICATION

(11) **EP 4 628 510 A1**
(43) Date of publication of application: **08.10.2025**
(21) Application number: 23896693.1
(22) Date of filing: 24.11.2023
(51) Int. Cl.: C07K 19/00, C07K 14/065, C12N 15/62, C12N 15/39, C12N 15/85, C12N 5/10, A61K 39/275, A61K 39/39, A61P 31/20

(54) **MULTI-ANTIGEN CHIMERIC POXVIRUS VACCINE AND USE THEREOF**

(30) Priority: 02.12.2022 CN 202211537702
(71) Applicant: Institute Of Microbiology, Chinese Academy Of Sciences, Beijing 100101 (CN)
(72) Inventor: GAO, Fu, Beijing 100101 (CN); WANG, Qihui, Beijing 100101 (CN); DU, Pei, Beijing 100101 (CN); KONG, Tianxiang, Beijing 100101 (CN); WU, Lili, Beijing 100101 (CN); QU, Xiao, Beijing 100101 (CN); QI, Jianxun, Beijing 100101 (CN); MA, Renyi, Beijing 100101 (CN)
(74) Representative: LLR
(86) International application number: PCT/CN2023/134141
(87) International publication number: WO 2024/114542

(57) **Abstract**

The present application relates to a multi-immunogen chimeric or mixed antigen for a poxvirus, particularly monkeypox virus, related products, and preparation method and use thereof. The chimeric or mixed antigen of the present application comprises three immunogens: (1) a monkeypox virus A35R protein or an antigenic fragment thereof (or a peptide fragment derived therefrom), (2) a monkeypox virus M1R protein or an antigenic fragment thereof (or a peptide fragment derived therefrom), and (3) a monkeypox virus B6R protein or an antigenic fragment thereof (or a peptide fragment derived therefrom), and can elicit an immune response against two types of infectious virus particles, namely intracellular mature virus particles (IMV) and extracellular envelope virus particles (EEV), thereby efficiently inducing specific immunoprotective effects against the monkeypox virus. Moreover, the poxvirus vaccine of the present application features good safety, rapid response, and scalable production capacity, thus showing excellent clinical application prospects.

## Description

### Cross-Reference to Related Applications

The present application claims priority to Chinese patent application No. 202211537702.4 filed on December 02, 2022 and entitled "Multi-Antigen Chimeric Vaccine Against Poxvirus and Use thereof", which is incorporated herein by reference in its entirety.

### Technical Field

The present application belongs to the field of biomedicine, and particularly relates to a multi-immunogen chimeric or mixed vaccine against a poxvirus and use thereof.

### Background Art

Poxviruses, represented by monkeypox virus, are a class of nucleocytoplasmic large DNA viruses. Their viral genomes are about 130-375 kbp in size, and can encode up to 200 viral proteins. In addition to the fact that the encoded viral proteins are complex, viral particles of poxviruses are also relatively complex, and have two types of infectious viral particles with different morphologies, called intracellular mature virus (IMV) and extracellular envelope virus (EEV), respectively. Among them, IMV, which has an envelope, has higher stability than EEV and is mainly involved in the virus transmission between hosts. EEV, which has a special outer membrane structure, is mainly involved in the spread of the virus within the host. Because IMV and EEV have different membrane structures, membrane components, and cell infection mechanisms, their surface neutralizing antigens are also completely different.

Monkeypox virus (MPXV), which is a poxvirus and is highly homologous to the smallpox virus, has been spreading locally in central and western Africa for a long time and has evolved multiple strains since its discovery in 1958. However, in 2022, MPXV showed a global epidemic trend. By July 23, it had spread to 75 countries and infected more than 15,000 people, and was thus declared a "public health emergency of international concern" by the World Health Organization. Although current vaccines provide defense against the monkeypox virus, it is worth noting that recent findings show that MPXV has a trend of increasing mutation frequency, and there is the possibility of producing escape mutant strains.

There are only two vaccines worldwide that can be used for preventing monkeypox virus infection, namely JYNNEOS ^{™} vaccine produced by Ankara-Bavarian Nordic (also known as Imvamune or Imvanex) and ACAM2000^{®} produced by Sanofi Pasteur. The two vaccines are attenuated live vaccines originally developed for preventing smallpox. ACAM2000^{®}, a second-generation vaccine, has replication capacity in the human body and thus brings risks such as encephalitis, myocarditis, and progressive vaccinia infection after vaccination, and is also unsuitable for vaccination of young children, pregnant women, and individuals with low or impaired immune function. By contrast, JYNNEOS^{™}, a third-generation vaccine, exhibits improved safety compared to the first-generation and second-generation vaccines since it cannot be replicated in the human body, but its immune efficacy is moderately reduced.

Live virus vaccines have the following common shortcomings: (1) the vaccines are derived from live viruses, and obtained by attenuated or weakened culture, and thereby they completely retain the antigens of the viruses, but there is the possibility of incomplete attenuation or restoration of virulence after mutation; (2) the vaccines contain all the antigens of the viruses, have complex components, and have a high possibility of causing side effects; (3) the proportion of effective immunogenic components in the vaccines is relatively low, resulting in weak immunogenicity even at the same dose of vaccination; (4) some viral components may inhibit host immunity, which will have a counterproductive effect and reduce the immunogenicity of the vaccine. Vaccines with defined components (e.g., mRNA vaccines/recombinant protein vaccines, etc.) can address the above problems. However, for complex viruses such as poxviruses, identifying key components to serve as immunogens is a major technical bottleneck.

The information disclosed in the Background section is intended solely to enhance understanding of the general background of the present application and shall not be construed as an acknowledgment or any form of suggestion that the information constitutes the prior art already known to those of ordinary skill in the art.

### Summary of the Invention

### Objective

In view of various drawbacks in the prior art vaccines, an objective of the present application is to provide a multi-immunogen chimeric or mixed poxvirus antigen which is capable of efficiently eliciting a specific immune response against poxviruses (especially monkeypox virus), related vaccine products, preparation method and use thereof. In addition, the vaccine products based on the chimeric or mixed antigen also have the advantages of safety, effectiveness, defined immunogenic components and protective mechanisms, high productivity, and low cost, so as to meet the safety and production capacity needs of large-scale vaccination of emergency vaccine populations.

### Solutions

In order to achieve the objective of the present application, the technical solutions provided by the present application are as follows:
In a first aspect, provided herein is a multi-immunogen chimeric or mixed poxvirus antigen comprising:
an immunogen I: a monkeypox virus A35R protein or an antigenic fragment thereof, or an amino acid sequence having at least 90%, 92%, 95%, 96%, 97%, 98%, or 99% identity thereto and having the same or substantially the same immunogenicity therewith;
an immunogen II: a monkeypox virus M1R protein or an antigenic fragment thereof, or an amino acid sequence having at least 90%, 92%, 95%, 96%, 97%, 98%, or 99% identity thereto and having the same or substantially the same immunogenicity therewith; and
an immunogen III: a monkeypox virus B6R protein or an antigenic fragment thereof, or an amino acid sequence having at least 90%, 92%, 95%, 96%, 97%, 98%, or 99% identity thereto and having the same or substantially the same immunogenicity therewith.

In a feasible implementation, the antigenic fragment of the A35R protein is an extracellular segment of the protein or a part thereof, preferably a peptide fragment having an amino acid sequence shown in SEQ ID NO: 1, or an amino acid sequence consisting of SEQ ID NO: 1 plus a fragment of 1 to 30 amino acids extending therefrom toward an N-terminus of the A35R protein, which is preferably an amino acid sequence shown in SEQ ID NO: 2;
and/or, the antigenic fragment of the M1R protein is an extracellular segment of the protein or a part thereof, preferably a peptide fragment having an amino acid sequence shown in SEQ ID NO: 3;
and/or, the antigenic fragment of the B6R protein is an extracellular segment of the protein or a part thereof, preferably a peptide fragment having an amino acid sequence shown in SEQ ID NO: 4.

In some embodiments, the multi-immunogen chimeric or mixed poxvirus antigen is a multi-immunogen mixed antigen which is a mixture of the immunogens I, II, and III;
wherein preferably, the immunogen I has an amino acid sequence shown in SEQ ID NO: 1 or SEQ ID NO: 2, or an amino acid sequence that is derived from the amino acid sequence shown in SEQ ID NO: 1 or SEQ ID NO: 2 by substitution, deletion, or addition of one or several amino acids and has the same or substantially the same immunogenicity therewith;
and/or, the immunogen II has an amino acid sequence shown in SEQ ID NO: 3, or an amino acid sequence that is derived from the amino acid sequence shown in SEQ ID NO: 3 by substitution, deletion, or addition of one or several amino acids and has the same or substantially the same immunogenicity therewith;
and/or, the immunogen III has an amino acid sequence shown in SEQ ID NO: 4, or an amino acid sequence that is derived from the amino acid sequence shown in SEQ ID NO: 4 by substitution, deletion, or addition of one or several amino acids and has the same or substantially the same immunogenicity therewith, which is preferably an amino acid sequence shown in SEQ ID NO: 5; preferably, a mass ratio of the immunogens I, II, III in the mixture is 1:1:1.

In some other embodiments, the multi-immunogen chimeric or mixed poxvirus antigen is a multi-immunogen chimeric antigen which is a single chain composed of one or more immunogens I, II, and III attached in tandem. In some particular embodiments, the chimeric antigen comprises an amino acid sequence arranged in a form of A1-C₁-M-C₂-A2-C₃-B, wherein:
A1 represents a monkeypox virus A35R protein or an antigenic fragment I thereof, or an amino acid sequence having at least 90%, 92%, 95%, 96%, 97%, 98%, or 99% identity thereto and having the same or substantially the same immunogenicity therewith,
A2 represents a monkeypox virus A35R protein or an antigenic fragment II thereof, or an amino acid sequence having at least 90%, 92%, 95%, 96%, 97%, 98%, or 99% identity thereto and having the same or substantially the same immunogenicity therewith,
M represents a monkeypox virus M1R protein or an antigenic fragment thereof, or an amino acid sequence having at least 90%, 92%, 95%, 96%, 97%, 98%, or 99% identity thereto and having the same or substantially the same immunogenicity therewith,
B represents a monkeypox virus B6R protein or an antigenic fragment thereof, or an amino acid sequence having at least 90%, 92%, 95%, 96%, 97%, 98%, or 99% identity thereto and having the same or substantially the same immunogenicity therewith,
C₁, C₂, and C₃ are each independently none, or a linker sequence (GGGGS)m, where m is any integer between 1 and 10; and,
wherein:
   A1 is the same as or different from A2.

In some preferred particular embodiments, the A1 represents an amino acid sequence shown in SEQ ID NO: 1, or an amino acid sequence that is derived from the amino acid sequence shown in SEQ ID NO: 1 by substitution, deletion, or addition of one or several amino acids and has the same or substantially the same immunogenicity therewith;
and/or, the A2 represents an amino acid sequence shown in SEQ ID NO: 1, or an amino acid sequence consisting of SEQ ID NO: 1 plus a fragment of 1 to 30 amino acids extending therefrom toward an N-terminus of the A35R protein, which is preferably an amino acid sequence shown in SEQ ID NO: 2, or an amino acid sequence that is derived from any of the above amino acid sequences by substitution, deletion, or addition of one or several amino acids and has the same or substantially the same immunogenicity therewith; wherein the A35R peptide fragment having the amino acid sequence shown in SEQ ID NO: 1 is a functional peptide fragment, while the fragment of 1 to 30 amino acids extending therefrom toward the N-terminus of the A35R protein serves as a linker;
and/or, the M represents an amino acid sequence shown in SEQ ID NO: 3, or an amino acid sequence that is derived from the amino acid sequence shown in SEQ ID NO: 3 by substitution, deletion, or addition of one or several amino acids and has the same or substantially the same immunogenicity therewith;
and/or, the B represents an amino acid sequence shown in SEQ ID NO: 4, or an amino acid sequence that is derived from the amino acid sequence shown in SEQ ID NO: 4 by substitution, deletion, or addition of one or several amino acids and has the same or substantially the same immunogenicity therewith, which is preferably an amino acid sequence shown in SEQ ID NO: 5.

Further preferably, the A1 represents the amino acid sequence shown in SEQ ID NO: 1, the A2 represents the amino acid sequence shown in SEQ ID NO: 1 or SEQ ID NO: 2, the M represents the amino acid sequence shown in SEQ ID NO: 3, and the B represents the amino acid sequence shown in SEQ ID NO: 4 or SEQ ID NO: 5.

In some preferred embodiments, the A1 represents the amino acid sequence shown in SEQ ID NO: 1, the A2 represents the amino acid sequence shown in SEQ ID NO: 2, the M represents the amino acid sequence shown in SEQ ID NO: 3, and the B represents the amino acid sequence shown in SEQ ID NO: 4 or SEQ ID NO: 5;
preferably, the C₁, C₂, C₃ are all none; and
further preferably, the chimeric antigen comprises an amino acid sequence shown in SEQ ID NO: 6 or SEQ ID NO: 7.

In some other particular embodiments, the chimeric antigen comprises an amino acid sequence arranged in a form of A-C₁'-M-C₂'- B, wherein:
A represents a monkeypox virus A35R protein or an antigenic fragment thereof, or an amino acid sequence having at least 90%, 92%, 95%, 96%, 97%, 98%, or 99% identity thereto and having the same or substantially the same immunogenicity therewith;
M represents a monkeypox virus M1R protein or an antigenic fragment thereof, or an amino acid sequence having at least 90%, 92%, 95%, 96%, 97%, 98%, or 99% identity thereto and having the same or substantially the same immunogenicity therewith,
B represents a monkeypox virus B6R protein or an antigenic fragment thereof, or an amino acid sequence having at least 90%, 92%, 95%, 96%, 97%, 98%, or 99% identity thereto and having the same or substantially the same immunogenicity therewith;
C₁' and C₂' are each independently none, or a linker sequence (GGGGS)n, where n is any integer between 1 and 10.

In some preferred particular embodiments, the A represents an amino acid sequence shown in SEQ ID NO: 1, or an amino acid sequence that is derived from the amino acid sequence shown in SEQ ID NO: 1 by substitution, deletion, or addition of one or several amino acids and has the same or substantially the same immunogenicity therewith;
and/or, the M represents an amino acid sequence shown in SEQ ID NO: 3, or an amino acid sequence that is derived from the amino acid sequence shown in SEQ ID NO: 3 by substitution, deletion, or addition of one or several amino acids and has the same or substantially the same immunogenicity therewith;
and/or, the B represents an amino acid sequence shown in SEQ ID NO: 4, or an amino acid sequence that is derived from the amino acid sequence shown in SEQ ID NO: 4 by substitution, deletion, or addition of one or several amino acids and has the same or substantially the same immunogenicity therewith, which is preferably an amino acid sequence shown in SEQ ID NO: 5.

SEQ ID NO: 5 comprises only one C140S mutation as compared to the sequence of SEQ ID NO: 4. After this mutation, the number of Cs (cysteines) on the surface of B6R peptide fragment changes from an odd number to an even number, such that the probability of protein forming multimers is reduced. This is because two cysteines on a peptide chain can form a disulfide bond; if there are an even number of cysteines on the peptide chain, they will tend to form intra-chain disulfide bonds; if there are an odd number of cysteines on the peptide chain, the excess one will bind to the excess cysteines on other peptide chains, thereby forming a multimer.

Further preferably, the A represents the amino acid sequence shown in SEQ ID NO: 1, the M represents the amino acid sequence shown in SEQ ID NO: 3, and the B represents the amino acid sequence shown in SEQ ID NO: 4 or SEQ ID NO: 5;
preferably, the C₁' and C₂' are both none; and
further preferably, the chimeric antigen comprises an amino acid sequence shown in SEQ ID NO: 8 or SEQ ID NO: 9.

Preferably, the chimeric antigen further comprises a signal peptide sequence at its N-terminus; and optionally, the signal peptide sequence is as shown in SEQ ID NO: 19.

In the present application, in order to take into account the immunoprotective effects against both types of virus particles EEV and IMV, a multi-immunogen chimeric or mixed antigen comprising both neutralizing antigens A35R and B6R of EEV and a neutralizing antigen M1R of IMV is designed, and the three neutralizing antigens are encoded by the A35R gene, the B6R gene, and the M1R gene of the monkeypox virus, respectively, which are homologous to vaccinia viruses A33R, B5R, and L1R, respectively.

In a second aspect, provided herein is a preparation method for the multi-immunogen chimeric poxvirus antigen of the first aspect above, comprising the steps of:
adding a Kozak sequence and a signal peptide coding sequence to a 5' terminus of the nucleotide sequence encoding the multi-immunogen chimeric poxvirus antigen of the first aspect, adding a His-tag coding sequence and a stop codon to a 3' terminus thereof, performing cloning and expression, selecting correct recombinants, then transfecting the correct recombinants into an expression system cell for expression, collecting a cell culture supernatant, and isolating the chimeric antigen therefrom.

In a feasible implementation of the above preparation method, the expression system cell is a mammalian cell, an insect cell, a yeast cell, or a bacterial cell;
optionally, the mammalian cell is an HEK293T cell, a 293F cell, or a CHO cell; further optionally, the 293F cell is an HEK293F cell, a Freestyle293F cell, or an Expi293F cell;
optionally, the insect cell is an sf9 cell, an Hi5 cell, an sf21 cell, or an S2 cell;
optionally, the yeast cell is a *Pichia pastoris* cell or a yeast cell modified therefrom; and optionally, the bacterial cell is an *Escherichia coli* cell.

In a third aspect, provided herein is a polynucleotide encoding the multi-immunogen chimeric or mixed poxvirus antigen of the first aspect.

In specific embodiments, the polynucleotide is a human codon-optimized nucleotide sequence, which can be DNA or mRNA;
when encoding the multi-immunogen chimeric poxvirus antigen, preferably, the polynucleotide is a DNA molecule comprising or consisting of a DNA sequence shown in one of SEQ ID NOs: 10, 11, 12, or 13; alternatively, the polynucleotide is an mRNA molecule comprising or consisting of an mRNA sequence shown in one of SEQ ID NOs: 14, 15, 16, or 17;
when encoding the multi-immunogen mixed poxvirus antigen, preferably, the polynucleotide is a polynucleotide group comprising or consisting of: a DNA molecule comprising a DNA sequence shown in SEQ ID NO: 22, a DNA molecule comprising a DNA sequence shown in SEQ ID NO: 23, and a DNA molecule comprising a DNA sequence shown in SEQ ID NO: 24; alternatively, the polynucleotide group comprises or consists of: an mRNA molecule comprising an mRNA sequence shown in SEQ ID NO: 25, an mRNA molecule comprising an mRNA sequence shown in SEQ ID NO: 26, and an mRNA molecule comprising an mRNA sequence shown in SEQ ID NO: 27.

In a fourth aspect, provided herein is a nucleic acid construct comprising the polynucleotide of the third aspect, and optionally at least one expression regulatory element operably linked to the polynucleotide.

In a fifth aspect, provided herein is an expression vector comprising the nucleic acid construct of the fourth aspect.

In a sixth aspect, provided herein is a host cell in which the polynucleotide of the third aspect, the nucleic acid construct of the fourth aspect, or the expression vector of the fifth aspect is transformed or transfected.

In a seventh aspect, provided herein is use of the multi-immunogen chimeric or mixed poxvirus antigen of the first aspect, the polynucleotide of the third aspect, the nucleic acid construct of the fourth aspect, the expression vector of the fifth aspect, or the host cell of the sixth aspect in the preparation of a medicament for the prevention and/treatment of poxvirus infections;
preferably, the poxvirus is selected from the group consisting of monkeypox virus, smallpox virus, cowpox virus, and/or vaccinia virus;
optionally, the medicament is a vaccine, for example, a DNA vaccine based on a eukaryotic expression vector or a viral vector, an mRNA vaccine, or a recombinant protein vaccine, and preferably the mRNA vaccine;
optionally, the vaccine is in a form of a nasal spray, an oral formulation, a suppository, or a parenteral formulation;
preferably, the nasal spray is selected from the group consisting of an aerosol, a spray, and a dry powder inhaler;
preferably, the oral formulation is selected from the group consisting of a tablet, a powder, a pill, granules, a soft/hard capsule, a film-coated agent, and an ointment; further preferably, the tablet is a sublingual tablet; further preferably, the granules are fine granules; further preferably, the powder is a pulvis; further preferably, the pellet is a pilule;
preferably, the parenteral formulation is a transdermal formulation, an ointment, a plaster, a topical liquid, or an injectable formulation; and further preferably, the injectable formulation is a formulation for infusion.

In an eighth aspect, provided herein is a vaccine or immunogenic composition comprising the multi-immunogen chimeric or mixed poxvirus antigen of the first aspect, the polynucleotide of the third aspect, the nucleic acid construct of the fourth aspect, the expression vector of the fifth aspect, or the host cell of the sixth aspect, and a physiologically acceptable vehicle, adjuvant, excipient, carrier, and/or diluent.

In some preferred particular embodiments, the vaccine or immunogenic composition is a recombinant protein vaccine against a poxvirus comprising the multi-immunogen chimeric or mixed poxvirus antigen of the first aspect and an adjuvant;
optionally, the adjuvant is one or more selected from the group consisting of an aluminum adjuvant, an MF59 adjuvant, and an MF59-like adjuvant.

In some other preferred particular embodiments, the vaccine or immunogenic composition is a DNA vaccine against a poxvirus, comprising:
(1) a eukaryotic expression vector; and
(2) a DNA sequence constructed into the eukaryotic expression vector and encoding the multi-immunogen chimeric or mixed poxvirus antigen of the first aspect;

preferably, when encoding the multi-immunogen chimeric poxvirus antigen, the DNA sequence constructed into the eukaryotic expression vector is a DNA sequence shown in one of SEQ ID NOs: 10, 11, 12, or 13;
preferably, when encoding the multi-immunogen mixed poxvirus antigen, the DNA vaccine comprises three eukaryotic expression constructs, with the DNA sequence in each of the eukaryotic expression constructs being shown in SEQ ID NOs: 22, 23, 24, respectively; optionally, the eukaryotic expression vector is selected from the group consisting of pGX0001, pVAX1, pCAGGS, and pcDNA series vectors.

In some other preferred particular embodiments, the vaccine or immunogenic composition is an mRNA vaccine against a poxvirus, comprising:
(I) an mRNA molecule encoding the multi-immunogen chimeric or mixed poxvirus antigen of the first aspect; and
(II) lipid nanoparticles;

preferably, when encoding the multi-immunogen chimeric poxvirus antigen, the mRNA molecule has an mRNA sequence shown in one of SEQ ID NOs: 14, 15, 16, or 17;
preferably, when encoding the multi-immunogen mixed poxvirus antigen, the mRNA vaccine comprises three mRNA molecules encapsulated within lipid nanoparticles having mRNA sequences shown in SEQ ID NOs: 25, 26, 27, respectively.

In some other preferred particular embodiments, the vaccine or immunogenic composition is a poxvirus-viral vector vaccine comprising:
(1) a viral backbone vector; and
(2) a DNA sequence constructed into the viral backbone vector and encoding the multi-immunogen chimeric or mixed poxvirus antigen of the first aspect;

preferably, when encoding the multi-immunogen chimeric poxvirus antigen, the DNA sequence constructed into the viral backbone vector is a DNA sequence shown in one of SEQ ID NOs: 10, 11, 12, or 13;
preferably, when encoding the multi-immunogen mixed poxvirus antigen, the poxvirus-viral vector vaccine comprises three viral vector constructs, with the DNA sequence in each of the viral vector constructs being shown in SEQ ID NOs: 22, 23, 24, respectively;
optionally, the viral backbone vector is one or more selected from the following viral vectors: an adenovirus vector, a poxvirus vector, an influenza virus vector, and an adeno-associated virus vector.

In a feasible implementation, the vaccine or immunogenic composition is in a form of a nasal spray, an oral formulation, a suppository, or a parenteral formulation;
preferably, the nasal spray is selected from the group consisting of an aerosol, a spray, and a dry powder inhaler;
preferably, the oral formulation is selected from the group consisting of a tablet, a powder, a pill, granules, a soft/hard capsule, a film-coated agent, and an ointment;
further preferably, the tablet is a sublingual tablet;
further preferably, the granules are fine granules;
further preferably, the powder is a pulvis;
further preferably, the pill is a pilule;
preferably, the parenteral formulation is a transdermal formulation, an ointment, a plaster, a topical liquid, or an injectable formulation; and further preferably, the injectable formulation is a formulation for infusion.

In a ninth aspect, provided herein is a method for preventing and/or treating poxvirus infections, comprising administering to a subject in need thereof a prophylactically and/or therapeutically effective amount of the following material: the multi-immunogen chimeric or mixed poxvirus antigen of the first aspect, the polynucleotide of the third aspect, the nucleic acid construct of the fourth aspect, the expression vector of the fifth aspect, the host cell of the sixth aspect, and/or the vaccine or immunogenic composition of the eighth aspect.

The expression "prophylactically and/or therapeutically effective amount" may vary depending on the subject administered, the subject organ, the symptoms, the method of administration, etc., and may be determined by the physician's judgment, taking into account the type of the dosage form, the method of administration, the age and weight and symptoms of the patient, etc.

### Beneficial Effects

The inventors of the present application have designed a multi-immunogen chimeric or mixed antigen against a poxvirus, particularly monkeypox virus, comprising the following three immunogens: (1) a monkeypox virus A35R protein or an antigenic fragment thereof (or a peptide fragment derived therefrom), (2) a monkeypox virus M1R protein or an antigenic fragment thereof (or a peptide fragment derived therefrom), and (3) a monkeypox virus B6R protein or an antigenic fragment thereof (or a peptide fragment derived therefrom), wherein the A35R and B6R are specific neutralizing antigens of intracellular mature virus particles (IMV), while the M1R is the specific neutralizing antigen of extracellular envelope virus particles (EEV). Vaccines comprising the three immunogens can stimulate an immune response against the two types of infectious virus particles.

In some specific embodiments according to the present application, a single-chain polypeptide is formed by attaching one or more of the monkeypox virus A35R protein or its antigenic fragment, the monkeypox virus M1R protein or its antigenic fragment, and the monkeypox virus B6R protein or its antigenic fragment in tandem directly or through an appropriate linker sequence, and the resulting single-chain polypeptide not only retains the immunogenicity of each of the three immunogens, but also can elicit specific protective antibodies against monkeypox virus more efficiently.

Compared with poxvirus vaccines in the prior art, the vaccine products based on the chimeric or mixed antigen of the present application have the following advantages:
1) The safety problems of the existing attenuated live virus vaccines can be overcome. Through experimental validation, the poxvirus vaccines of the present application have good effectiveness, safety, and a rapid response.
2) They have high specificity for the monkeypox virus. The existing live virus vaccines are primarily developed based on vaccinia viruses. Although the vaccinia viruses and the monkeypox virus belong to the same Poxviridae family, there are still certain differences in their neutralizing antigen sequences, and therefore, their protective effect against the monkeypox virus remains unclear. However, the poxvirus vaccines of the present application are developed based on the epitope of the monkeypox virus, and thus have high specificity for the prevention and/or treatment of the monkeypox virus.
3) There are many kinds of proteins in the monkeypox virus, and most of the proteins cannot stimulate an effective antiviral immune response, and thus are ineffective components. In addition, there are also some viral proteins having immunosuppressive effects. The existing live virus vaccine cannot remove the above-mentioned ineffective and detrimental components, so there are vaccination risks and uncertainties. However, the poxvirus vaccines of the present application have no components that are either ineffective or detrimental to eliciting an effective immune response in the host in the original immunogen, thereby avoiding the inherent safety issues associated with attenuated vaccines. Furthermore, the experimental data demonstrate that the chimeric or mixed vaccine comprising only the aforementioned three immunogens in the present application can exhibit complete protective effects in mouse models.

### Brief Description of the Drawings

One or more examples are exemplified by the pictures in the accompanying drawings that correspond thereto and are not intended to be limiting of the embodiments. As used herein, the word "exemplary" means "serving as an instance or an example, or illustrative". Any example described herein as "exemplary" is not necessarily to be construed as superior or better than other examples.
Figure 1 shows the expression effect of a constructed chimeric mRNA in cells as detected by Western Blot, as recited in Example 1 of the present application.
Figure 2 shows a schematic diagram of an immunization and serum sampling procedure employed in Example 2 of the present application.
Figure 3 shows the antibody titer detection results for specific binding antibodies against A35R, M1R, and B6R antigen proteins in mouse sera collected on Day 13 (Figure 3a) and Day 27 (Figure 3b) of each chimeric mRNA vaccine immunization procedure as detected by enzyme-linked immunosorbent assay (ELISA), as recited in Example 3 of the present application.
Figure 4 shows the plaque neutralization results of mouse sera collected on Day 27 of each chimeric mRNA vaccine immunization procedure as assayed in Example 4 of the present application.
Figure 5 shows the protective efficacy of each chimeric mRNA vaccine against VACV-WR intranasal challenge in BALB/c mice as assayed in Example 5 of the present application, wherein the abscissa shows the number of days post-viral challenge, and the ordinate shows the percentage change in body weight (a) and the percentage survival (b) of mice.
Figure 6 shows the protective efficacy of the mixed mRNA vaccine "A/M/B" against VACV-WR intranasal challenge in BALB/c mice as assayed in Example 5 of the present application, wherein the abscissa shows the number of days post-viral challenge, and the ordinate shows the percentage change in body weight (a) and the percentage survival (b) of mice.

### Detailed Description of the Invention

In order to make the objective, technical solutions and advantages of the present application clearer, the technical solutions in the examples of the present application will be described clearly and completely, obviously, the described examples are some of the examples of the present application, but not all of them. Based on the examples of the present application, all other examples obtained by those of ordinary skill in the art without creative work are within the scope of the present application.

In addition, in order to better explain the present application, a lot of specific details are given in the following particular embodiments. It will be understood by those skilled in the art that the present application may be practiced without certain specific details. In some examples, materials, elements, methods, means, etc., well known to those skilled in the art, are not described in detail so as to highlight the spirit of the present application.

Throughout the specification and claims, the term "comprise" or variations thereof, such as "comprising" or "comprised", will be understood to include the stated components and not to exclude other elements or other components, unless expressly indicated otherwise.

### Example 1: Antigen design, in vitro preparation, intracellular expression test, and lipid nanoparticle encapsulation of multi-immunogen chimeric or mixed mRNA vaccines against a poxvirus

### Antigen design of mRNA vaccines:

In this example, the following chimeric mRNA vaccines were designed and prepared as representative examples of the present application:
"AMAB": in an arrangement of A35R-M1R-A35R-B6R in sequence from N-terminus to C-terminus, in which the amino acid sequence of the first A35R peptide fragment starting from the N-terminus was shown in SEQ ID NO: 1, the amino acid sequence of the second A35R peptide fragment starting from the N-terminus was shown in SEQ ID NO: 2, the amino acid sequence of M1R was shown in SEQ ID NO: 3, and the amino acid sequence of B6R was shown in SEQ ID NO: 4;
"AMAB-C140S": in an arrangement of A35R-M1R-A35R-B6R' in sequence from N-terminus to C-terminus, in which the amino acid sequence of the first A35R peptide fragment starting from the N-terminus was shown in SEQ ID NO: 1, the amino acid sequence of the second A35R peptide fragment starting from the N-terminus was shown in SEQ ID NO: 2, the amino acid sequence of M1R was shown in SEQ ID NO: 3, and the amino acid sequence of B6R' was shown in SEQ ID NO: 5;
"AMB-C140S": in an arrangement of A35R- M1R-B6R' in sequence from N-terminus to C-terminus, in which the amino acid sequence of A35R was shown in SEQ ID NO: 1; the amino acid sequence of M1R was shown in SEQ ID NO: 3, and the amino acid sequence of B6R' was shown in SEQ ID NO: 5.

In addition, in this example, a mixed vaccine of A35R, M1R, and B6R mRNA vaccines (hereinafter referred to as "A/M/B") was designed and prepared. Specifically, mRNA vaccines of A35R, M1R, and B6R single immunogens were prepared and encapsulated, respectively, and the three vaccines were mixed in a mass ratio of 1:1:1; wherein the A35R, M1R, and B6R single immunogens were the A35R peptide fragment shown in SEQ ID NO: 1, the M1R peptide fragment shown in SEQ ID NO: 3, and the B6R peptide fragment shown in SEQ ID NO: 4, respectively.

### In vitro preparation, intracellular expression test, and lipid nanoparticle encapsulation

### 1) In vitro transcription and capping of mRNA vaccines

In this example, the basic plasmid used for in vitro transcription of mRNA vaccines was pUC57, which was provided by Nanjing GenScript Biotechnology Co., Ltd.

DNA expression elements of mRNA vaccines were introduced into the basic plasmid pUC57 by conventional molecular biological means. The expression elements comprised: (1) a T7 promoter; (2) DNA coding region of mRNA vaccines (the DNA coding sequences of chimeric mRNA vaccines "AMAB", "AMAB-C140S", "AMB-C140S" were as shown in SEQ ID NOs: 10, 11, and 13, respectively, and the DNA coding sequences of single mRNA vaccines "A35R", "M1R", "B6R" were as shown in SEQ ID NOs: 22, 23, and 24, respectively); (3) 5' UTR sequence (as shown in SEQ ID NO: 18) upstream of the coding region; (4) the coding sequence (i.e., SP, as shown in SEQ ID NO: 20) of the signal peptide (as shown in SEQ ID NO: 19); (5) 3' UTR sequence (as shown in SEQ ID NO: 21) downstream of the coding region, and the polyadenosine tail (Poly-A-tail).

First, for in vitro transcription of each mRNA, the plasmids described above were digested using the restriction endonuclease BamHI for linearization; purification was performed using a conventional DNA purification method to obtain a template for in vitro transcription; then, based on the template, in vitro transcription was performed using a T7RNA In Vitro Transcription Kit (E131-01A, Suzhou Novoprotein Scientific Co., Ltd.) to obtain the in vitro transcribed mRNA; finally, the mRNA was purified by the lithium chloride precipitation method using a Lithium Chloride Recovery Kit (S125, Suzhou Novoprotein Scientific Co., Ltd.) to obtain each purified in vitro transcribed mRNA.

Then, by using a Cap1 Capping Enzyme Kit (M082-01B, Suzhou Novoprotein Scientific Co., Ltd.), the purified in vitro transcribed mRNA was subjected to 5'-end Cap1 capping to facilitate translation in eukaryotic cells; afterwards, the mRNA was re-purified using the aforementioned lithium chloride precipitation method to obtain each purified 5'-capped mRNA.

### 2) Intracellular expression assay of chimeric mRNA

The mRNAs obtained from step 1) were subjected to intracellular expression analysis by the following procedures:

### (1) mRNA transfection

HEK293T cells were plated in a 12-well plate such that the cell density reached about 50% the next day; 1 µg of mRNAs of AMAB, AMAB-C140S, or AMB-C140S were added to 100 µL of serum-free Opti-MEM together with a TransIT-mRNA reagent (2 µL) and a booster reagent (2 µL). The mixture was incubated for 3 minutes, and then added dropwise to the 12-well plate; and 36 hours after transfection, cells were harvested.

### (2) Western Blot

The cells obtained in step (1) were lysed with a cell lysis buffer, and then the cell lysate was mixed with a loading buffer containing dithiothreitol (DTT). The mixture was separated by 10% SDS-PAGE, followed by membrane transfer to a PVDF membrane. Then, the PVDF membrane was blocked in 5% skim milk, incubated with mouse serum immunized with AMAB-C140S as the primary antibody and goat anti-mouse IgG-HRP (EASYBio) as the secondary antibody for one hour each. Finally, color development was performed using the Beyotime Beyo ECL Plus color development solution.

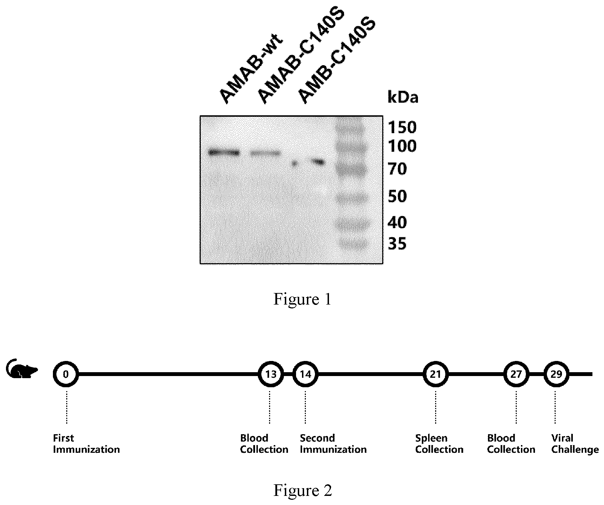

The Western Blot results were shown in Figure 1, which showed the expression of each mRNA in cells. As can be seen from Figure 1, AMAB, AMAB-C140S, and AMB-C140 mRNAs were successfully expressed in cells, and their expression products exhibited a single band, indicating that they were single immunogens.

### 3) Encapsulation of mRNAs with lipid nanoparticles (LNPs)

A cationic lipid, phosphatidylcholine, cholesterol, and PEG lipid were mixed in a ratio of 50:10:38.5:1.5, and then mixed with each 5'-capped mRNA (including "AMAB", "AMAB-C140S", "AMB-C140S" chimeric mRNAs, and "A35R", "M1R", and "B6R" single mRNAs) for encapsulation using a Nanoassemblr Benchtop nanoliposome formulation system manufactured by Precision Nano Systems. Following encapsulation, the buffer solution was exchanged for PBS by centrifugation or dialysis. The mRNA encapsulation efficiency was determined using a Quan-iT Ribogreen RNA reagent kit from Thermo Fisher and the results showed that the encapsulation efficiency met the requirements for mRNA vaccines.

To prepare the mixed vaccine "A/M/B" composed of A35R, M1R, and B6R mRNAs, the "A35R", "M1R", and "B6R" single mRNA vaccines prepared and encapsulated by the above methods were mixed in a mass ratio of 1:1:1.

### Example 2: Immunization of experimental animals and sample collection

In this example, animal experiments were performed using BALB/c female mice aged 6-8 weeks (purchased from Weitonglihua Experimental Animal Co., Ltd). The experimental groups were divided into mRNA vaccine immunization groups and a negative control group, wherein the mRNA vaccine immunization groups included AMAB, AMAB-C140S, and AMB-C140S mRNA vaccine immunization groups, and the negative control group was LNP immunization group.

All mice in the mRNA vaccine immunization groups were immunized with one dose of the chimeric mRNA vaccines prepared in Example 1, namely the AMAB, AMAB-C140S, or AMB-C140S mRNA vaccine, or the mixed mRNA vaccine "A/M/B" prepared in Example 1, on Day 0 and Day 14, respectively. Mice in the negative control group were injected with the same dose of empty LNP at the same time points. Vaccinations were administered via intramuscular injection at a dose of 7.5 µg mRNA vaccine or empty LNP per mouse at a time. Mouse serum samples were collected on Day 13 and Day 27, respectively, to determine the binding antibody titers and pseudovirus neutralization antibody titers, respectively. In addition, mouse spleen samples were collected on Day 21 to access T cell-mediated immune responses.

The mRNA vaccine immunization of mice and the sampling procedure were shown in Figure 2.

### Example 3: Detection of specific binding antibody titers in mouse sera immunized with poxvirus chimeric mRNA vaccines

An ELISA plate was coated with 0.2 µg/ml monkeypox virus A35R, M1R, or B6R antigen protein (the amino acid sequences thereof were shown in SEQ ID NOs: 1, 3, and 4, respectively), and then blocked with 5% skim milk for 1 hour. Then, sera collected from mice in each experimental group in Example 2 were incubated at 56°C for 30 minutes for inactivation. The inactivated serum samples were diluted in a three-fold gradient starting from 1:200 or 1:1000, then the dilution was added to each well, and the ELISA plate was subsequently incubated at 37 °C for 1 hour. Goat anti-mouse IgG-HRP antibody (purchased from EASYBio) was added to the plate as a secondary antibody and the plate was incubated at 37 °C for another 1 hour. Finally, 3,3',5,5'-tetramethylbenzidine (TMB) substrate was added for color development. After the color development was complete, the reaction was terminated with 2 M hydrochloric acid, and the absorbances at 450 nm and 630 nm were measured using a microplate reader (PerkinElmer). The absorbance value was calculated by subtracting the absorbance at 630 nm from that at 450 nm of the same well. The endpoint titer was defined as the corresponding dilution factor of serum when the absorbance produced by serum (the absorbance at 450 nm minus that at 630 nm as described above) was 2.1 times greater than the background value. An antibody titer below the detection limit was defined as one third of the detection limit.

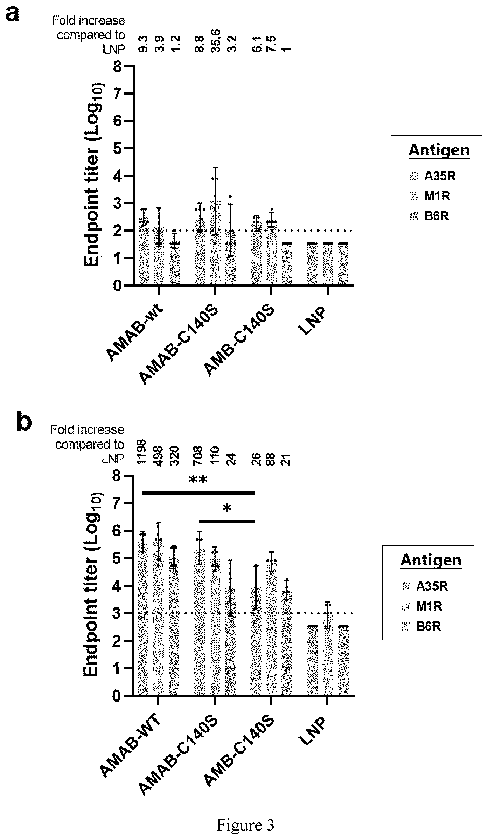

The antibody titer detection results of immunized mouse sera were shown in Figure 3, where the number above each bar represented the fold increase in the geometric mean compared to that of the corresponding antigen in the LNP group. Figure 3 showed that the sera after the first immunization (a) and the second immunization (b) of the three mRNA vaccines all contained specific binding antibodies against A35, M1, and B6 antigen proteins, and the antibody titer of the serum after the second immunization was increased by 1 to 2 orders of magnitude compared with that after the first immunization.

The above results demonstrated that AMAB, AMAB-C140S, or AMB-C140S mRNA vaccines all induced high levels of specific binding antibodies against the A35R, M1R, and B6R antigen proteins.

### Example 4: Plaque neutralization assay with immunized mouse sera

Vaccinia virus (VACV), the model virus of the Orthopoxvirus genus, is an orthopoxvirus that can be handled in standard BSL-2 laboratories. In view of the minimal interspecies difference between VACV and other orthopoxvirus species, VACV is commonly used internationally to evaluate orthopoxvirus vaccines at the cellular and animal levels. Therefore, we used a mouse-adapted vaccinia virus strain Western Reserve (VACV-WR), which was frequently used internationally, to access the live virus neutralizing capacity of mRNA vaccines at the cellular level.

Specifically, Vero cells were plated in a 12-well plate, and one day later, the immunized mouse serum collected on Day 27 of the immunization procedure in Example 2 was serially diluted 10 times in a deep well plate with 2% FBS DMEM medium in a 2-fold gradient. An equal volume of 100 PFU of VACV virus was mixed with the diluted serum and incubated at 37°C for 1 hour. The cell culture supernatant was discarded and a serum-virus mixture was added to incubate at 37°C for 2 hours. The supernatant was discarded, the resultant was rinsed once with PBS, and the cells were overlaid with a 1:1 mixture of sodium carboxymethylcellulose and 2x DMEM, and cultured at 37°C for 48 hours. 4% paraformaldehyde was added to fix the cells at room temperature for 3 hours, and the mixture was discarded. Following staining with crystal violet for 120 minutes, plaques were counted using an ELISpot counter. Inhibition rate curve was plotted according to the reduction in plaque numbers compared to the positive control across different serum dilutions, and the IC₅₀ (half-maximal inhibitory concentration) value of the curve was defined as the PRNT₅₀ titer value of the serum sample.

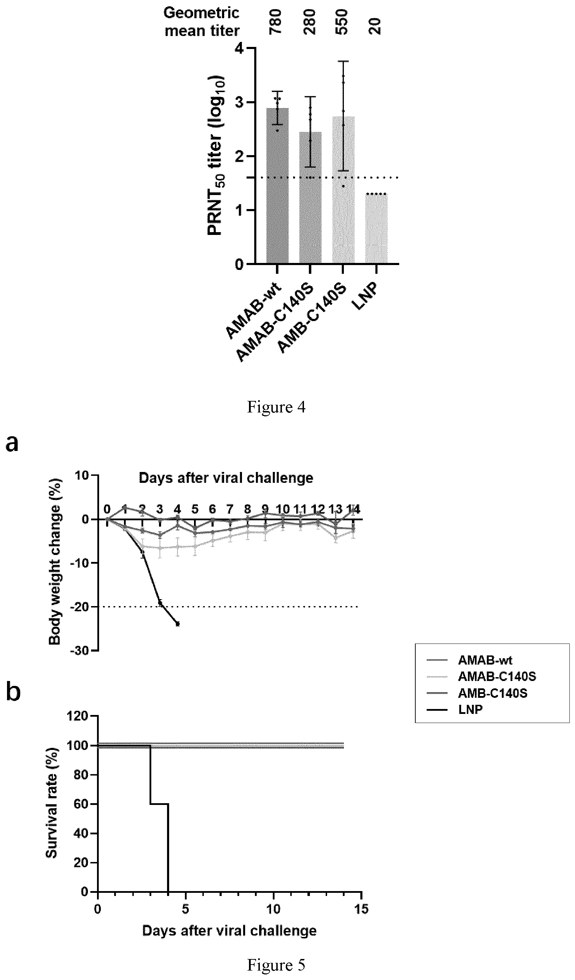

The results were shown in Figure 4, which showed that AMAB, AMAB-C140S, or AMB-C140S mRNA vaccines all induced high levels of neutralizing antibodies against monkeypox virus, with GMTs (geometric mean titers) being 780, 280, and 550, respectively.

### Example 5: Challenge protection assay

Based on the inventors' preliminary experimental, all mice died 7 days after infection when challenged with 1.9 × 10⁵ PFU of VACV-WR, and therefore, this viral dose was used as the challenge dose for animal experiments in this example.

In this example, mice in the chimeric mRNA vaccine immunization group, the mixed mRNA vaccine immunization group, and the LNP immunization group in Example 2 were subjected to VACV-WR intranasal challenge, respectively, on Day 29 after the first immunization, with the challenge dose being 1.9 × 10⁵ PFU. By observing the survival rate and body weight changes of mice in the mRNA vaccine immunization groups and the negative control group, the protective efficacy of each mRNA vaccine against live virus challenge in mice was evaluated.

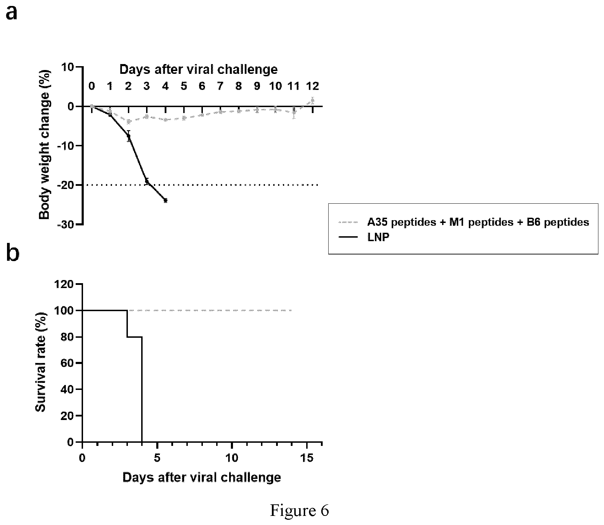

The results were shown in Figures 5 and 6.

Figure 5 showed that the chimeric mRNA vaccines AMAB, AMAB-C140S, and AMB-C140S of the present application provided 100% protective efficacy against VACV-WR infection in mice, with no significant body weight loss. In contrast, the mice in the unimmunized group showed continuous body weight loss within 4 days until they all died.

Figure 6 showed that the mixed mRNA vaccine (i.e., "A/M/B") composed of A35R peptide fragment, M1R peptide fragment, and B6R peptide fragment of the present application also provided 100% protective efficacy against VACV-WR infection in mice, with no significant body weight loss or slight loss followed by rapid recovery. In contrast, the unimmunized mice exhibited continuous body weight loss within 4 days until they all died.

To sum up, the multi-antigen chimeric vaccine of the present application can efficiently elicit a specific immune response against a poxvirus (particularly the monkeypox virus), and thus is highly expected to be developed into a preventive and/or therapeutic vaccine product against a poxvirus (particularly the monkeypox virus), demonstrating great clinical application prospects.

Finally, it should be noted that the above embodiments are only used to illustrate, rather than to limit, the technical solutions of the present application . Although the present application has been described in detail with reference to the foregoing embodiments, it will be understood by one of ordinary skill in the art that the technical solutions described in the foregoing embodiments can still be modified or some technical features can be equivalently substituted. These modifications or substitutions do not make the essence of the corresponding technical solutions depart from the spirit and scope of the present application.

### Industrial Applicability

The multi-immunogen chimeric or mixed poxvirus antigen provided by the present application can elicit an immune response against two types of infectious virus particles, namely intracellular mature virus particles (IMV) and extracellular envelope virus particles (EEV), thereby efficiently inducing specific immunoprotective effects against the monkeypox virus. Moreover, the poxvirus vaccine of the present application features good safety, rapid response, and scalable production capacity, thus showing excellent clinical application prospects.

### The sequences involved in the present application:

SEQ ID NO: 1 (the amino acid sequence of A35R antigenic fragment I) 
STTQYDHKESCNGLYYQGSCYILHSDYKSFEDAKANCAAESSTLPNKSDVLTTWLIDYVE
DTWGSDGNPITKTTSDYQDSDVSQEVRKYFCT
SEQ ID NO: 2 (the amino acid sequence of A35R antigenic fragment II) 
SANKAAITDSAVAVAAASSTHRKVVSSTTQYDHKESCNGLYYQGSCYILHSDYKSFEDAK
ANCAAESSTLPNKSDVLTTWLIDYVEDTWGSDGNPITKTTSDYQDSDVSQEVRKYFCT
SEQ ID NO: 3 (the amino acid sequence of M1R peptide fragment) 
AAASIQTTVNTLSERISSKLEQEANASAQTKCDIEIGNFYIRQNHGCNITVKNMCSADADA
QLDAVLSAATETYSGLTPEQKAYVPAMFTAALNIQTSVNTVVRDFENYVKQTCNSSAVVD
NKLKIQNVIIDECYGAPGSPTNLEFINTGSSKGNCAIKALMQLTTKATTQIAPRQVAG
SEQ ID NO: 4 (the amino acid sequence of B6R peptide fragment) 
TCTVPTMNNAKLTSTETSFNDKQKVTFTCDSGYHSLDPNAVCETDKWKYENPCKKMCTV
SDYVSELYDKPLYEVNSTMTLSCNGETKYFRCEEKNGNTSWNDTVTCPNAECQPLQLEH
GSCQPVKEKYSFGEYMTINCDVGYEVIGVSYISCTANSWNVIPSCQQKCDIPSLSNGLISGS
TFSIGGVIHLSCKSGFTLTGSPSSTCIDGKWNPILPTCVRSNEEFDPVDDGPDDETDLSKLSK
DVVQYEQEIESLEATYH
SEQ ID NO: 5 (the amino acid sequence of B6R peptide fragment containing C140S mutation) 
TCTVPTMNNAKLTSTETSFNDKQKVTFTCDSGYHSLDPNAVCETDKWKYENPCKKMCTV
SDYVSELYDKPLYEVNSTMTLSCNGETKYFRCEEKNGNTSWNDTVTCPNAECQPLQLEH
GSSQPVKEKYSFGEYMTINCDVGYEVIGVSYISCTANSWNVIPSCQQKCDIPSLSNGLISGS
TFSIGGVIHLSCKSGFTLTGSPSSTCIDGKWNPILPTCVRSNEEFDPVDDGPDDETDLSKLSK
DVVQYEQEIESLEATYH
SEQ ID NO: 6 (the amino acid sequence of AMAB) 
STTQYDHKESCNGLYYQGSCYILHSDYKSFEDAKANCAAESSTLPNKSDVLTTWLIDYVE
DTWGSDGNPITKTTSDYQDSDVSQEVRKYFCTAAASIQTTVNTLSERISSKLEQEANASAQ
 
TKCDIEIGNFYIRQNHGCNITVKNMCSADADAQLDAVLSAATETYSGLTPEQKAYVPAMFT
AALNIQTSVNTVVRDFENYVKQTCNSSAVVDNKLKIQNVIIDECYGAPGSPTNLEFINTGS
SKGNCAIKALMQLTTKATTQIAPRQVAGSANKAAITDSAVAVAAASSTHRKVVSSTTQYD
HKESCNGLYYQGSCYILHSDYKSFEDAKANCAAESSTLPNKSDVLTTWLIDYVEDTWGSD
GNPITKTTSDYQDSDVSQEVRKYFCTTCTVPTMNNAKLTSTETSFNDKQKVTFTCDSGYH
SLDPNAVCETDKWKYENPCKKMCTVSDYVSELYDKPLYEVNSTMTLSCNGETKYFRCEE
KNGNTSWNDTVTCPNAECQPLQLEHGSCQPVKEKYSFGEYMTINCDVGYEVIGVSYISCT
ANSWNVIPSCQQKCDIPSLSNGLISGSTFSIGGVIHLSCKSGFTLTGSPSSTCIDGKWNPILPT
CVRSNEEFDPVDDGPDDETDLSKLSKDVVQYEQEIESLEATYH
SEQ ID NO: 7 (the amino acid sequence of AMAB-C140S) 
STTQYDHKESCNGLYYQGSCYILHSDYKSFEDAKANCAAESSTLPNKSDVLTTWLIDYVE
DTWGSDGNPITKTTSDYQDSDVSQEVRKYFCTAAASIQTTVNTLSERISSKLEQEANASAQ
TKCDIEIGNFYIRQNHGCNITVKNMCSADADAQLDAVLSAATETYSGLTPEQKAYVPAMFT
AALNIQTSVNTVVRDFENYVKQTCNSSAVVDNKLKIQNVIIDECYGAPGSPTNLEFINTGS
SKGNCAIKALMQLTTKATTQIAPRQVAGSANKAAITDSAVAVAAASSTHRKVVSSTTQYD
HKESCNGLYYQGSCYILHSDYKSFEDAKANCAAESSTLPNKSDVLTTWLIDYVEDTWGSD
GNPITKTTSDYQDSDVSQEVRKYFCTTCTVPTMNNAKLTSTETSFNDKQKVTFTCDSGYH
SLDPNAVCETDKWKYENPCKKMCTVSDYVSELYDKPLYEVNSTMTLSCNGETKYFRCEE
KNGNTSWNDTVTCPNAECQPLQLEHGSSQPVKEKYSFGEYMTINCDVGYEVIGVSYISCT
ANSWNVIPSCQQKCDIPSLSNGLISGSTFSIGGVIHLSCKSGFTLTGSPSSTCIDGKWNPILPT
CVRSNEEFDPVDDGPDDETDLSKLSKDVVQYEQEIESLEATYH
SEQ ID NO: 8 (the amino acid sequence of AMB) 
STTQYDHKESCNGLYYQGSCYILHSDYKSFEDAKANCAAESSTLPNKSDVLTTWLIDYVE
DTWGSDGNPITKTTSDYQDSDVSQEVRKYFCTAAASIQTTVNTLSERISSKLEQEANASAQ
TKCDIEIGNFYIRQNHGCNITVKNMCSADADAQLDAVLSAATETYSGLTPEQKAYVPAMFT
AALNIQTSVNTVVRDFENYVKQTCNSSAVVDNKLKIQNVIIDECYGAPGSPTNLEFINTGS
SKGNCAIKALMQLTTKATTQIAPRQVAGTCTVPTMNNAKLTSTETSFNDKQKVTFTCDSG
YHSLDPNAVCETDKWKYENPCKKMCTVSDYVSELYDKPLYEVNSTMTLSCNGETKYFRC
EEKNGNTSWNDTVTCPNAECQPLQLEHGSCQPVKEKYSFGEYMTINCDVGYEVIGVSYIS
 
CTANSWNVIPSCQQKCDIPSLSNGLISGSTFSIGGVIHLSCKSGFTLTGSPSSTCIDGKWNPIL
PTCVRSNEEFDPVDDGPDDETDLSKLSKDVVQYEQEIESLEATYH
SEQ ID NO: 9 (the amino acid sequence of AMB-C140S) 
STTQYDHKESCNGLYYQGSCYILHSDYKSFEDAKANCAAESSTLPNKSDVLTTWLIDYVE
DTWGSDGNPITKTTSDYQDSDVSQEVRKYFCTAAASIQTTVNTLSERISSKLEQEANASAQ
TKCDIEIGNFYIRQNHGCNITVKNMCSADADAQLDAVLSAATETYSGLTPEQKAYVPAMFT
AALNIQTSVNTVVRDFENYVKQTCNSSAVVDNKLKIQNVIIDECYGAPGSPTNLEFINTGS
SKGNCAIKALMQLTTKATTQIAPRQVAGTCTVPTMNNAKLTSTETSFNDKQKVTFTCDSG
YHSLDPNAVCETDKWKYENPCKKMCTVSDYVSELYDKPLYEVNSTMTLSCNGETKYFRC
EEKNGNTSWNDTVTCPNAECQPLQLEHGSSQPVKEKYSFGEYMTINCDVGYEVIGVSYIS
CTANSWNVIPSCQQKCDIPSLSNGLISGSTFSIGGVIHLSCKSGFTLTGSPSSTCIDGKWNPIL
PTCVRSNEEFDPVDDGPDDETDLSKLSKDVVQYEQEIESLEATYH
SEQ ID NO: 10 (the DNA sequence of AMAB) 
AGCACCACCCAGTACGACCACAAAGAGAGCTGCAACGGCCTGTACTACCAAGGCTCT
TGTTACATCCTGCACAGCGACTATAAGAGCTTCGAGGATGCTAAGGCCAATTGCGCCGC
CGAGAGCAGCACACTGCCTAACAAGTCCGACGTGCTGACCACCTGGCTGATCGACTAC
GTGGAAGATACATGGGGAAGCGACGGCAACCCCATCACAAAAACCACAAGCGATTATC
AGGACAGCGACGTGTCTCAGGAGGTTAGAAAGTACTTTTGCACCGCCGCCGCCTCTAT
CCAGACCACAGTGAACACCCTGAGCGAGCGGATCAGCTCCAAGCTGGAGCAGGAGGC
AAACGCAAGCGCCCAGACAAAGTGCGACATCGAGATCGGCAATTTCTACATCAGACA
GAACCACGGCTGCAATATCACCGTGAAGAATATGTGCTCCGCCGACGCAGATGCACAG
CTGGATGCCGTGCTGTCCGCCGCAACCGAGACATACTCTGGCCTGACCCCTGAGCAGA
AGGCCTATGTGCCAGCCATGTTCACAGCCGCCCTGAACATCCAGACCAGCGTGAATAC
AGTGGTGCGGGACTTTGAGAACTACGTGAAGCAGACCTGCAATTCTAGCGCCGTGGTG
GACAACAAGCTGAAGATCCAGAATGTGATCATCGATGAGTGTTATGGAGCACCAGGCT
CCCCTACCAACCTGGAGTTCATCAATACAGGCTCCTCTAAGGGCAACTGCGCCATCAA
GGCCCTGATGCAGCTGACCACAAAGGCCACCACACAGATCGCACCTAGGCAGGTGGC
AGGCTCCGCCAATAAGGCAGCCATCACCGACTCTGCCGTGGCAGTGGCAGCAGCCAG
CTCCACACACCGCAAGGTGGTGTCTAGCACCACACAGTACGATCACAAGGAATCCTGT
 
AATGGCCTGTACTATCAGGGCTCCTGTTACATCCTGCACTCTGACTATAAGAGCTTTGA
GGATGCCAAGGCCAACTGTGCCGCCGAGTCCTCTACCCTGCCAAATAAGAGCGACGTG
CTGACCACATGGCTGATCGACTATGTGGAGGATACCTGGGGCTCTGATGGCAACCCCAT
CACAAAGACCACATCTGATTACCAGGACAGCGACGTGAGCCAGGAGGTGCGGAAGTA
TTTCTGCACCACGTGCACGGTGCCCACGATGAACAACGCGAAGTTGACGTCCACGGA
AACCTCTTTCAATGATAAACAAAAAGTCACGTTCACATGTGACTCCGGATACCACTCTT
TGGACCCGAACGCAGTTTGTGAAACAGATAAGTGGAAGTACGAGAATCCGTGCAAGA
AAATGTGCACGGTGTCGGATTATGTATCAGAGTTGTACGACAAGCCGTTGTACGAGGTT
AATAGTACCATGACCTTGTCGTGCAACGGCGAAACCAAGTATTTCCGTTGTGAAGAGA
AAAACGGGAACACGTCGTGGAACGACACGGTAACCTGCCCCAACGCGGAGTGTCAAC
CTCTGCAGTTGGAGCACGGCTCTTGTCAGCCGGTGAAGGAAAAATATTCCTTCGGCGA
GTACATGACTATAAACTGTGATGTGGGGTACGAGGTTATAGGCGTATCCTACATTAGCTG
CACCGCTAATAGTTGGAACGTCATCCCCTCGTGCCAGCAGAAATGCGACATTCCGTCTC
TGTCAAATGGCCTGATCTCCGGGTCGACCTTCTCGATAGGTGGGGTTATTCATTTGTCCT
GTAAGAGTGGGTTCACTCTTACAGGCTCCCCATCTTCGACCTGCATAGATGGGAAGTG
GAACCCCATCCTTCCCACTTGTGTCAGGTCGAACGAGGAATTCGATCCGGTAGATGAC
GGCCCTGACGACGAGACGGATCTGTCGAAGCTGTCCAAAGACGTGGTTCAGTATGAG
CAGGAGATAGAATCTCTTGAGGCTACTTACCAC
SEQ ID NO: 11 (the DNA sequence of AMAB-C140S) 
AGCACCACCCAGTACGACCACAAAGAGAGCTGCAACGGCCTGTACTACCAAGGCTCT
TGTTACATCCTGCACAGCGACTATAAGAGCTTCGAGGATGCTAAGGCCAATTGCGCCGC
CGAGAGCAGCACACTGCCTAACAAGTCCGACGTGCTGACCACCTGGCTGATCGACTAC
GTGGAAGATACATGGGGAAGCGACGGCAACCCCATCACAAAAACCACAAGCGATTATC
AGGACAGCGACGTGTCTCAGGAGGTTAGAAAGTACTTTTGCACCGCCGCCGCCTCTAT
CCAGACCACAGTGAACACCCTGAGCGAGCGGATCAGCTCCAAGCTGGAGCAGGAGGC
AAACGCAAGCGCCCAGACAAAGTGCGACATCGAGATCGGCAATTTCTACATCAGACA
GAACCACGGCTGCAATATCACCGTGAAGAATATGTGCTCCGCCGACGCAGATGCACAG
CTGGATGCCGTGCTGTCCGCCGCAACCGAGACATACTCTGGCCTGACCCCTGAGCAGA
AGGCCTATGTGCCAGCCATGTTCACAGCCGCCCTGAACATCCAGACCAGCGTGAATAC
AGTGGTGCGGGACTTTGAGAACTACGTGAAGCAGACCTGCAATTCTAGCGCCGTGGTG
 
GACAACAAGCTGAAGATCCAGAATGTGATCATCGATGAGTGTTATGGAGCACCAGGCT
CCCCTACCAACCTGGAGTTCATCAATACAGGCTCCTCTAAGGGCAACTGCGCCATCAA
GGCCCTGATGCAGCTGACCACAAAGGCCACCACACAGATCGCACCTAGGCAGGTGGC
AGGCTCCGCCAATAAGGCAGCCATCACCGACTCTGCCGTGGCAGTGGCAGCAGCCAG
CTCCACACACCGCAAGGTGGTGTCTAGCACCACACAGTACGATCACAAGGAATCCTGT
AATGGCCTGTACTATCAGGGCTCCTGTTACATCCTGCACTCTGACTATAAGAGCTTTGA
GGATGCCAAGGCCAACTGTGCCGCCGAGTCCTCTACCCTGCCAAATAAGAGCGACGTG
CTGACCACATGGCTGATCGACTATGTGGAGGATACCTGGGGCTCTGATGGCAACCCCAT
CACAAAGACCACATCTGATTACCAGGACAGCGACGTGAGCCAGGAGGTGCGGAAGTA
TTTCTGCACCACGTGCACGGTGCCCACGATGAACAACGCGAAGTTGACGTCCACGGA
AACCTCTTTCAATGATAAACAAAAAGTCACGTTCACATGTGACTCCGGATACCACTCTT
TGGACCCGAACGCAGTTTGTGAAACAGATAAGTGGAAGTACGAGAATCCGTGCAAGA
AAATGTGCACGGTGTCGGATTATGTATCAGAGTTGTACGACAAGCCGTTGTACGAGGTT
AATAGTACCATGACCTTGTCGTGCAACGGCGAAACCAAGTATTTCCGTTGTGAAGAGA
AAAACGGGAACACGTCGTGGAACGACACGGTAACCTGCCCCAACGCGGAGTGTCAAC
CTCTGCAGTTGGAGCACGGCTCTTCTCAGCCGGTGAAGGAAAAATATTCCTTCGGCGA
GTACATGACTATAAACTGTGATGTGGGGTACGAGGTTATAGGCGTATCCTACATTAGCTG
CACCGCTAATAGTTGGAACGTCATCCCCTCGTGCCAGCAGAAATGCGACATTCCGTCTC
TGTCAAATGGCCTGATCTCCGGGTCGACCTTCTCGATAGGTGGGGTTATTCATTTGTCCT
GTAAGAGTGGGTTCACTCTTACAGGCTCCCCATCTTCGACCTGCATAGATGGGAAGTG
GAACCCCATCCTTCCCACTTGTGTCAGGTCGAACGAGGAATTCGATCCGGTAGATGAC
GGCCCTGACGACGAGACGGATCTGTCGAAGCTGTCCAAAGACGTGGTTCAGTATGAG
CAGGAGATAGAATCTCTTGAGGCTACTTACCAC
SEQ ID NO: 12 (the DNA sequence of AMB) 
AGCACCACCCAGTACGACCACAAAGAGAGCTGCAACGGCCTGTACTACCAAGGCTCT
TGTTACATCCTGCACAGCGACTATAAGAGCTTCGAGGATGCTAAGGCCAATTGCGCCGC
CGAGAGCAGCACACTGCCTAACAAGTCCGACGTGCTGACCACCTGGCTGATCGACTAC
GTGGAAGATACATGGGGAAGCGACGGCAACCCCATCACAAAAACCACAAGCGATTATC
AGGACAGCGACGTGTCTCAGGAGGTTAGAAAGTACTTTTGCACCGCCGCCGCCTCTAT
CCAGACCACAGTGAACACCCTGAGCGAGCGGATCAGCTCCAAGCTGGAGCAGGAGGC
 
AAACGCAAGCGCCCAGACAAAGTGCGACATCGAGATCGGCAATTTCTACATCAGACA
GAACCACGGCTGCAATATCACCGTGAAGAATATGTGCTCCGCCGACGCAGATGCACAG
CTGGATGCCGTGCTGTCCGCCGCAACCGAGACATACTCTGGCCTGACCCCTGAGCAGA
AGGCCTATGTGCCAGCCATGTTCACAGCCGCCCTGAACATCCAGACCAGCGTGAATAC
AGTGGTGCGGGACTTTGAGAACTACGTGAAGCAGACCTGCAATTCTAGCGCCGTGGTG
GACAACAAGCTGAAGATCCAGAATGTGATCATCGATGAGTGTTATGGAGCACCAGGCT
CCCCTACCAACCTGGAGTTCATCAATACAGGCTCCTCTAAGGGCAACTGCGCCATCAA
GGCCCTGATGCAGCTGACCACAAAGGCCACCACACAGATCGCACCTAGGCAGGTGGC
AGGCACGTGCACGGTGCCCACGATGAACAACGCGAAGTTGACGTCCACGGAAACCTC
TTTCAATGATAAACAAAAAGTCACGTTCACATGTGACTCCGGATACCACTCTTTGGACC
CGAACGCAGTTTGTGAAACAGATAAGTGGAAGTACGAGAATCCGTGCAAGAAAATGT
GCACGGTGTCGGATTATGTATCAGAGTTGTACGACAAGCCGTTGTACGAGGTTAATAGT
ACCATGACCTTGTCGTGCAACGGCGAAACCAAGTATTTCCGTTGTGAAGAGAAAAACG
GGAACACGTCGTGGAACGACACGGTAACCTGCCCCAACGCGGAGTGTCAACCTCTGC
AGTTGGAGCACGGCTCTTGTCAGCCGGTGAAGGAAAAATATTCCTTCGGCGAGTACAT
GACTATAAACTGTGATGTGGGGTACGAGGTTATAGGCGTATCCTACATTAGCTGCACCG
CTAATAGTTGGAACGTCATCCCCTCGTGCCAGCAGAAATGCGACATTCCGTCTCTGTCA
AATGGCCTGATCTCCGGGTCGACCTTCTCGATAGGTGGGGTTATTCATTTGTCCTGTAA
GAGTGGGTTCACTCTTACAGGCTCCCCATCTTCGACCTGCATAGATGGGAAGTGGAAC
CCCATCCTTCCCACTTGTGTCAGGTCGAACGAGGAATTCGATCCGGTAGATGACGGCC
CTGACGACGAGACGGATCTGTCGAAGCTGTCCAAAGACGTGGTTCAGTATGAGCAGG
AGATAGAATCTCTTGAGGCTACTTACCAC
SEQ ID NO: 13 (the DNA sequence of AMB-C140S) 
AGCACCACCCAGTACGACCACAAAGAGAGCTGCAACGGCCTGTACTACCAAGGCTCT
TGTTACATCCTGCACAGCGACTATAAGAGCTTCGAGGATGCTAAGGCCAATTGCGCCGC
CGAGAGCAGCACACTGCCTAACAAGTCCGACGTGCTGACCACCTGGCTGATCGACTAC
GTGGAAGATACATGGGGAAGCGACGGCAACCCCATCACAAAAACCACAAGCGATTATC
AGGACAGCGACGTGTCTCAGGAGGTTAGAAAGTACTTTTGCACCGCCGCCGCCTCTAT
CCAGACCACAGTGAACACCCTGAGCGAGCGGATCAGCTCCAAGCTGGAGCAGGAGGC
AAACGCAAGCGCCCAGACAAAGTGCGACATCGAGATCGGCAATTTCTACATCAGACA
 
GAACCACGGCTGCAATATCACCGTGAAGAATATGTGCTCCGCCGACGCAGATGCACAG
CTGGATGCCGTGCTGTCCGCCGCAACCGAGACATACTCTGGCCTGACCCCTGAGCAGA
AGGCCTATGTGCCAGCCATGTTCACAGCCGCCCTGAACATCCAGACCAGCGTGAATAC
AGTGGTGCGGGACTTTGAGAACTACGTGAAGCAGACCTGCAATTCTAGCGCCGTGGTG
GACAACAAGCTGAAGATCCAGAATGTGATCATCGATGAGTGTTATGGAGCACCAGGCT
CCCCTACCAACCTGGAGTTCATCAATACAGGCTCCTCTAAGGGCAACTGCGCCATCAA
GGCCCTGATGCAGCTGACCACAAAGGCCACCACACAGATCGCACCTAGGCAGGTGGC
AGGCACGTGCACGGTGCCCACGATGAACAACGCGAAGTTGACGTCCACGGAAACCTC
TTTCAATGATAAACAAAAAGTCACGTTCACATGTGACTCCGGATACCACTCTTTGGACC
CGAACGCAGTTTGTGAAACAGATAAGTGGAAGTACGAGAATCCGTGCAAGAAAATGT
GCACGGTGTCGGATTATGTATCAGAGTTGTACGACAAGCCGTTGTACGAGGTTAATAGT
ACCATGACCTTGTCGTGCAACGGCGAAACCAAGTATTTCCGTTGTGAAGAGAAAAACG
GGAACACGTCGTGGAACGACACGGTAACCTGCCCCAACGCGGAGTGTCAACCTCTGC
AGTTGGAGCACGGCTCTTCTCAGCCGGTGAAGGAAAAATATTCCTTCGGCGAGTACAT
GACTATAAACTGTGATGTGGGGTACGAGGTTATAGGCGTATCCTACATTAGCTGCACCG
CTAATAGTTGGAACGTCATCCCCTCGTGCCAGCAGAAATGCGACATTCCGTCTCTGTCA
AATGGCCTGATCTCCGGGTCGACCTTCTCGATAGGTGGGGTTATTCATTTGTCCTGTAA
GAGTGGGTTCACTCTTACAGGCTCCCCATCTTCGACCTGCATAGATGGGAAGTGGAAC
CCCATCCTTCCCACTTGTGTCAGGTCGAACGAGGAATTCGATCCGGTAGATGACGGCC
CTGACGACGAGACGGATCTGTCGAAGCTGTCCAAAGACGTGGTTCAGTATGAGCAGG
AGATAGAATCTCTTGAGGCTACTTACCAC
SEQ ID NO: 14 (the mRNA sequence of AMAB) 
AGCACCACCCAGUACGACCACAAAGAGAGCUGCAACGGCCUGUACUACCAAGGCUC
UUGUUACAUCCUGCACAGCGACUAUAAGAGCUUCGAGGAUGCUAAGGCCAAUUGC
GCCGCCGAGAGCAGCACACUGCCUAACAAGUCCGACGUGCUGACCACCUGGCUGAU
CGACUACGUGGAAGAUACAUGGGGAAGCGACGGCAACCCCAUCACAAAAACCACAA
GCGAUUAUCAGGACAGCGACGUGUCUCAGGAGGUUAGAAAGUACUUUUGCACCGC
CGCCGCCUCUAUCCAGACCACAGUGAACACCCUGAGCGAGCGGAUCAGCUCCAAGC
UGGAGCAGGAGGCAAACGCAAGCGCCCAGACAAAGUGCGACAUCGAGAUCGGCAA
UUUCUACAUCAGACAGAACCACGGCUGCAAUAUCACCGUGAAGAAUAUGUGCUCC
 
GCCGACGCAGAUGCACAGCUGGAUGCCGUGCUGUCCGCCGCAACCGAGACAUACUC
UGGCCUGACCCCUGAGCAGAAGGCCUAUGUGCCAGCCAUGUUCACAGCCGCCCUGA
ACAUCCAGACCAGCGUGAAUACAGUGGUGCGGGACUUUGAGAACUACGUGAAGCA
GACCUGCAAUUCUAGCGCCGUGGUGGACAACAAGCUGAAGAUCCAGAAUGUGAUC
AUCGAUGAGUGUUAUGGAGCACCAGGCUCCCCUACCAACCUGGAGUUCAUCAAUAC
AGGCUCCUCUAAGGGCAACUGCGCCAUCAAGGCCCUGAUGCAGCUGACCACAAAGG
CCACCACACAGAUCGCACCUAGGCAGGUGGCAGGCUCCGCCAAUAAGGCAGCCAUC
ACCGACUCUGCCGUGGCAGUGGCAGCAGCCAGCUCCACACACCGCAAGGUGGUGUC
UAGCACCACACAGUACGAUCACAAGGAAUCCUGUAAUGGCCUGUACUAUCAGGGC
UCCUGUUACAUCCUGCACUCUGACUAUAAGAGCUUUGAGGAUGCCAAGGCCAACU
GUGCCGCCGAGUCCUCUACCCUGCCAAAUAAGAGCGACGUGCUGACCACAUGGCUG
AUCGACUAUGUGGAGGAUACCUGGGGCUCUGAUGGCAACCCCAUCACAAAGACCAC
AUCUGAUUACCAGGACAGCGACGUGAGCCAGGAGGUGCGGAAGUAUUUCUGCACC
ACGUGCACGGUGCCCACGAUGAACAACGCGAAGUUGACGUCCACGGAAACCUCUUU
CAAUGAUAAACAAAAAGUCACGUUCACAUGUGACUCCGGAUACCACUCUUUGGAC
CCGAACGCAGUUUGUGAAACAGAUAAGUGGAAGUACGAGAAUCCGUGCAAGAAAA
UGUGCACGGUGUCGGAUUAUGUAUCAGAGUUGUACGACAAGCCGUUGUACGAGGU
UAAUAGUACCAUGACCUUGUCGUGCAACGGCGAAACCAAGUAUUUCCGUUGUGAA
GAGAAAAACGGGAACACGUCGUGGAACGACACGGUAACCUGCCCCAACGCGGAGU
GUCAACCUCUGCAGUUGGAGCACGGCUCUUGUCAGCCGGUGAAGGAAAAAUAUUC
CUUCGGCGAGUACAUGACUAUAAACUGUGAUGUGGGGUACGAGGUUAUAGGCGUA
UCCUACAUUAGCUGCACCGCUAAUAGUUGGAACGUCAUCCCCUCGUGCCAGCAGAA
AUGCGACAUUCCGUCUCUGUCAAAUGGCCUGAUCUCCGGGUCGACCUUCUCGAUAG
GUGGGGUUAUUCAUUUGUCCUGUAAGAGUGGGUUCACUCUUACAGGCUCCCCAUC
UUCGACCUGCAUAGAUGGGAAGUGGAACCCCAUCCUUCCCACUUGUGUCAGGUCGA
ACGAGGAAUUCGAUCCGGUAGAUGACGGCCCUGACGACGAGACGGAUCUGUCGAA
GCUGUCCAAAGACGUGGUUCAGUAUGAGCAGGAGAUAGAAUCUCUUGAGGCUACU
UACCAC
SEQ ID NO:15 (the mRNA sequence of AMAB-C140S) AGCACCACCCAGUACGACCACAAAGAGAGCUGCAACGGCCUGUACUACCAAGGCUC 
UUGUUACAUCCUGCACAGCGACUAUAAGAGCUUCGAGGAUGCUAAGGCCAAUUGC
GCCGCCGAGAGCAGCACACUGCCUAACAAGUCCGACGUGCUGACCACCUGGCUGAU
CGACUACGUGGAAGAUACAUGGGGAAGCGACGGCAACCCCAUCACAAAAACCACAA
GCGAUUAUCAGGACAGCGACGUGUCUCAGGAGGUUAGAAAGUACUUUUGCACCGC
CGCCGCCUCUAUCCAGACCACAGUGAACACCCUGAGCGAGCGGAUCAGCUCCAAGC
UGGAGCAGGAGGCAAACGCAAGCGCCCAGACAAAGUGCGACAUCGAGAUCGGCAA
UUUCUACAUCAGACAGAACCACGGCUGCAAUAUCACCGUGAAGAAUAUGUGCUCC
GCCGACGCAGAUGCACAGCUGGAUGCCGUGCUGUCCGCCGCAACCGAGACAUACUC
UGGCCUGACCCCUGAGCAGAAGGCCUAUGUGCCAGCCAUGUUCACAGCCGCCCUGA
ACAUCCAGACCAGCGUGAAUACAGUGGUGCGGGACUUUGAGAACUACGUGAAGCA
GACCUGCAAUUCUAGCGCCGUGGUGGACAACAAGCUGAAGAUCCAGAAUGUGAUC
AUCGAUGAGUGUUAUGGAGCACCAGGCUCCCCUACCAACCUGGAGUUCAUCAAUAC
AGGCUCCUCUAAGGGCAACUGCGCCAUCAAGGCCCUGAUGCAGCUGACCACAAAGG
CCACCACACAGAUCGCACCUAGGCAGGUGGCAGGCUCCGCCAAUAAGGCAGCCAUC
ACCGACUCUGCCGUGGCAGUGGCAGCAGCCAGCUCCACACACCGCAAGGUGGUGUC
UAGCACCACACAGUACGAUCACAAGGAAUCCUGUAAUGGCCUGUACUAUCAGGGC
UCCUGUUACAUCCUGCACUCUGACUAUAAGAGCUUUGAGGAUGCCAAGGCCAACU
GUGCCGCCGAGUCCUCUACCCUGCCAAAUAAGAGCGACGUGCUGACCACAUGGCUG
AUCGACUAUGUGGAGGAUACCUGGGGCUCUGAUGGCAACCCCAUCACAAAGACCAC
AUCUGAUUACCAGGACAGCGACGUGAGCCAGGAGGUGCGGAAGUAUUUCUGCACC
ACGUGCACGGUGCCCACGAUGAACAACGCGAAGUUGACGUCCACGGAAACCUCUUU
CAAUGAUAAACAAAAAGUCACGUUCACAUGUGACUCCGGAUACCACUCUUUGGAC
CCGAACGCAGUUUGUGAAACAGAUAAGUGGAAGUACGAGAAUCCGUGCAAGAAAA
UGUGCACGGUGUCGGAUUAUGUAUCAGAGUUGUACGACAAGCCGUUGUACGAGGU
UAAUAGUACCAUGACCUUGUCGUGCAACGGCGAAACCAAGUAUUUCCGUUGUGAA
GAGAAAAACGGGAACACGUCGUGGAACGACACGGUAACCUGCCCCAACGCGGAGU
GUCAACCUCUGCAGUUGGAGCACGGCUCUUCUCAGCCGGUGAAGGAAAAAUAUUC
CUUCGGCGAGUACAUGACUAUAAACUGUGAUGUGGGGUACGAGGUUAUAGGCGUA
UCCUACAUUAGCUGCACCGCUAAUAGUUGGAACGUCAUCCCCUCGUGCCAGCAGAA
AUGCGACAUUCCGUCUCUGUCAAAUGGCCUGAUCUCCGGGUCGACCUUCUCGAUAG
GUGGGGUUAUUCAUUUGUCCUGUAAGAGUGGGUUCACUCUUACAGGCUCCCCAUC
 
UUCGACCUGCAUAGAUGGGAAGUGGAACCCCAUCCUUCCCACUUGUGUCAGGUCGA
ACGAGGAAUUCGAUCCGGUAGAUGACGGCCCUGACGACGAGACGGAUCUGUCGAA
GCUGUCCAAAGACGUGGUUCAGUAUGAGCAGGAGAUAGAAUCUCUUGAGGCUACU
UACCAC
SEQ ID NO: 16 (the mRNA sequence of AMB) 
AGCACCACCCAGUACGACCACAAAGAGAGCUGCAACGGCCUGUACUACCAAGGCUC
UUGUUACAUCCUGCACAGCGACUAUAAGAGCUUCGAGGAUGCUAAGGCCAAUUGC
GCCGCCGAGAGCAGCACACUGCCUAACAAGUCCGACGUGCUGACCACCUGGCUGAU
CGACUACGUGGAAGAUACAUGGGGAAGCGACGGCAACCCCAUCACAAAAACCACAA
GCGAUUAUCAGGACAGCGACGUGUCUCAGGAGGUUAGAAAGUACUUUUGCACCGC
CGCCGCCUCUAUCCAGACCACAGUGAACACCCUGAGCGAGCGGAUCAGCUCCAAGC
UGGAGCAGGAGGCAAACGCAAGCGCCCAGACAAAGUGCGACAUCGAGAUCGGCAA
UUUCUACAUCAGACAGAACCACGGCUGCAAUAUCACCGUGAAGAAUAUGUGCUCC
GCCGACGCAGAUGCACAGCUGGAUGCCGUGCUGUCCGCCGCAACCGAGACAUACUC
UGGCCUGACCCCUGAGCAGAAGGCCUAUGUGCCAGCCAUGUUCACAGCCGCCCUGA
ACAUCCAGACCAGCGUGAAUACAGUGGUGCGGGACUUUGAGAACUACGUGAAGCA
GACCUGCAAUUCUAGCGCCGUGGUGGACAACAAGCUGAAGAUCCAGAAUGUGAUC
AUCGAUGAGUGUUAUGGAGCACCAGGCUCCCCUACCAACCUGGAGUUCAUCAAUAC
AGGCUCCUCUAAGGGCAACUGCGCCAUCAAGGCCCUGAUGCAGCUGACCACAAAGG
CCACCACACAGAUCGCACCUAGGCAGGUGGCAGGCACGUGCACGGUGCCCACGAUG
AACAACGCGAAGUUGACGUCCACGGAAACCUCUUUCAAUGAUAAACAAAAAGUCA
CGUUCACAUGUGACUCCGGAUACCACUCUUUGGACCCGAACGCAGUUUGUGAAACA
GAUAAGUGGAAGUACGAGAAUCCGUGCAAGAAAAUGUGCACGGUGUCGGAUUAUG
UAUCAGAGUUGUACGACAAGCCGUUGUACGAGGUUAAUAGUACCAUGACCUUGUC
GUGCAACGGCGAAACCAAGUAUUUCCGUUGUGAAGAGAAAAACGGGAACACGUCG
UGGAACGACACGGUAACCUGCCCCAACGCGGAGUGUCAACCUCUGCAGUUGGAGCA
CGGCUCUUGUCAGCCGGUGAAGGAAAAAUAUUCCUUCGGCGAGUACAUGACUAUA
AACUGUGAUGUGGGGUACGAGGUUAUAGGCGUAUCCUACAUUAGCUGCACCGCUA
AUAGUUGGAACGUCAUCCCCUCGUGCCAGCAGAAAUGCGACAUUCCGUCUCUGUCA
AAUGGCCUGAUCUCCGGGUCGACCUUCUCGAUAGGUGGGGUUAUUCAUUUGUCCU
 
GUAAGAGUGGGUUCACUCUUACAGGCUCCCCAUCUUCGACCUGCAUAGAUGGGAA
GUGGAACCCCAUCCUUCCCACUUGUGUCAGGUCGAACGAGGAAUUCGAUCCGGUAG
AUGACGGCCCUGACGACGAGACGGAUCUGUCGAAGCUGUCCAAAGACGUGGUUCA
GUAUGAGCAGGAGAUAGAAUCUCUUGAGGCUACUUACCAC
SEQ ID NO: 17 (the mRNA sequence of AMB-C140S) 
AGCACCACCCAGUACGACCACAAAGAGAGCUGCAACGGCCUGUACUACCAAGGCUC
UUGUUACAUCCUGCACAGCGACUAUAAGAGCUUCGAGGAUGCUAAGGCCAAUUGC
GCCGCCGAGAGCAGCACACUGCCUAACAAGUCCGACGUGCUGACCACCUGGCUGAU
CGACUACGUGGAAGAUACAUGGGGAAGCGACGGCAACCCCAUCACAAAAACCACAA
GCGAUUAUCAGGACAGCGACGUGUCUCAGGAGGUUAGAAAGUACUUUUGCACCGC
CGCCGCCUCUAUCCAGACCACAGUGAACACCCUGAGCGAGCGGAUCAGCUCCAAGC
UGGAGCAGGAGGCAAACGCAAGCGCCCAGACAAAGUGCGACAUCGAGAUCGGCAA
UUUCUACAUCAGACAGAACCACGGCUGCAAUAUCACCGUGAAGAAUAUGUGCUCC
GCCGACGCAGAUGCACAGCUGGAUGCCGUGCUGUCCGCCGCAACCGAGACAUACUC
UGGCCUGACCCCUGAGCAGAAGGCCUAUGUGCCAGCCAUGUUCACAGCCGCCCUGA
ACAUCCAGACCAGCGUGAAUACAGUGGUGCGGGACUUUGAGAACUACGUGAAGCA
GACCUGCAAUUCUAGCGCCGUGGUGGACAACAAGCUGAAGAUCCAGAAUGUGAUC
AUCGAUGAGUGUUAUGGAGCACCAGGCUCCCCUACCAACCUGGAGUUCAUCAAUAC
AGGCUCCUCUAAGGGCAACUGCGCCAUCAAGGCCCUGAUGCAGCUGACCACAAAGG
CCACCACACAGAUCGCACCUAGGCAGGUGGCAGGCACGUGCACGGUGCCCACGAUG
AACAACGCGAAGUUGACGUCCACGGAAACCUCUUUCAAUGAUAAACAAAAAGUCA
CGUUCACAUGUGACUCCGGAUACCACUCUUUGGACCCGAACGCAGUUUGUGAAACA
GAUAAGUGGAAGUACGAGAAUCCGUGCAAGAAAAUGUGCACGGUGUCGGAUUAUG
UAUCAGAGUUGUACGACAAGCCGUUGUACGAGGUUAAUAGUACCAUGACCUUGUC
GUGCAACGGCGAAACCAAGUAUUUCCGUUGUGAAGAGAAAAACGGGAACACGUCG
UGGAACGACACGGUAACCUGCCCCAACGCGGAGUGUCAACCUCUGCAGUUGGAGCA
CGGCUCUUCUCAGCCGGUGAAGGAAAAAUAUUCCUUCGGCGAGUACAUGACUAUA
AACUGUGAUGUGGGGUACGAGGUUAUAGGCGUAUCCUACAUUAGCUGCACCGCUA
AUAGUUGGAACGUCAUCCCCUCGUGCCAGCAGAAAUGCGACAUUCCGUCUCUGUCA
AAUGGCCUGAUCUCCGGGUCGACCUUCUCGAUAGGUGGGGUUAUUCAUUUGUCCU
 
GUAAGAGUGGGUUCACUCUUACAGGCUCCCCAUCUUCGACCUGCAUAGAUGGGAA
GUGGAACCCCAUCCUUCCCACUUGUGUCAGGUCGAACGAGGAAUUCGAUCCGGUAG
AUGACGGCCCUGACGACGAGACGGAUCUGUCGAAGCUGUCCAAAGACGUGGUUCA
GUAUGAGCAGGAGAUAGAAUCUCUUGAGGCUACUUACCAC
SEQ ID NO: 18 (the 5' UTR sequence)
   GGGAAATAAGAGAGAAAAGAAGAGTAAGAAGAAATATAAGAGCCACC
SEQ ID NO: 19 (the signal peptide sequence)
   METDTLLLWVLLLWVPGSTG
SEQ ID NO: 20 (the coding sequence of the signal peptide) 
ATGGAAACAGACACCCTGCTGCTGTGGGTCCTGCTGCTCTGGGTGCCTGGCAGCACCG
GC
SEQ ID NO: 21 (the 3' UTR sequence) 
TGATAATAGGCTGGAGCCTCGGTGGCCATGCTTCTTGCCCCTTGGGCCTCCCCCCAGCC
CCTCCTCCCCTTCCTGCACCCGTACCCCCGTGGTCTTTGAATAAAGTCTGA
SEQ ID NO: 22 (the DNA sequence of A35R peptide fragment) 
AGCACCACCCAGTACGACCACAAAGAGAGCTGCAACGGCCTGTACTACCAAGGCTCT
TGTTACATCCTGCACAGCGACTATAAGAGCTTCGAGGATGCTAAGGCCAATTGCGCCGC
CGAGAGCAGCACACTGCCTAACAAGTCCGACGTGCTGACCACCTGGCTGATCGACTAC
GTGGAAGATACATGGGGAAGCGACGGCAACCCCATCACAAAAACCACAAGCGATTATC
AGGACAGCGACGTGTCTCAGGAGGTTAGAAAGTACTTTTGCACC
SEQ ID NO: 23 (the DNA sequence of M1R peptide fragment) 
GCCGCCGCCTCTATCCAGACCACAGTGAACACCCTGAGCGAGCGGATCAGCTCCAAGC
TGGAGCAGGAGGCAAACGCAAGCGCCCAGACAAAGTGCGACATCGAGATCGGCAATT
TCTACATCAGACAGAACCACGGCTGCAATATCACCGTGAAGAATATGTGCTCCGCCGA
CGCAGATGCACAGCTGGATGCCGTGCTGTCCGCCGCAACCGAGACATACTCTGGCCTG
 
ACCCCTGAGCAGAAGGCCTATGTGCCAGCCATGTTCACAGCCGCCCTGAACATCCAGA
CCAGCGTGAATACAGTGGTGCGGGACTTTGAGAACTACGTGAAGCAGACCTGCAATTC
TAGCGCCGTGGTGGACAACAAGCTGAAGATCCAGAATGTGATCATCGATGAGTGTTAT
GGAGCACCAGGCTCCCCTACCAACCTGGAGTTCATCAATACAGGCTCCTCTAAGGGCA
ACTGCGCCATCAAGGCCCTGATGCAGCTGACCACAAAGGCCACCACACAGATCGCAC
CTAGGCAGGTGGCAGGC
SEQ ID NO: 24 (the DNA sequence of B6R peptide fragment) 
ACGTGCACGGTGCCCACGATGAACAACGCGAAGTTGACGTCCACGGAAACCTCTTTC
AATGATAAACAAAAAGTCACGTTCACATGTGACTCCGGATACCACTCTTTGGACCCGA
ACGCAGTTTGTGAAACAGATAAGTGGAAGTACGAGAATCCGTGCAAGAAAATGTGCA
CGGTGTCGGATTATGTATCAGAGTTGTACGACAAGCCGTTGTACGAGGTTAATAGTACC
ATGACCTTGTCGTGCAACGGCGAAACCAAGTATTTCCGTTGTGAAGAGAAAAACGGG
AACACGTCGTGGAACGACACGGTAACCTGCCCCAACGCGGAGTGTCAACCTCTGCAG
TTGGAGCACGGCTCTTGTCAGCCGGTGAAGGAAAAATATTCCTTCGGCGAGTACATGA
CTATAAACTGTGATGTGGGGTACGAGGTTATAGGCGTATCCTACATTAGCTGCACCGCTA
ATAGTTGGAACGTCATCCCCTCGTGCCAGCAGAAATGCGACATTCCGTCTCTGTCAAAT
GGCCTGATCTCCGGGTCGACCTTCTCGATAGGTGGGGTTATTCATTTGTCCTGTAAGAG
TGGGTTCACTCTTACAGGCTCCCCATCTTCGACCTGCATAGATGGGAAGTGGAACCCCA
TCCTTCCCACTTGTGTCAGGTCGAACGAGGAATTCGATCCGGTAGATGACGGCCCTGA
CGACGAGACGGATCTGTCGAAGCTGTCCAAAGACGTGGTTCAGTATGAGCAGGAGATA
GAATCTCTTGAGGCTACTTACCAC
SEQ ID NO: 25 (the mRNA sequence of A35R peptide fragment) 
AGCACCACCCAGUACGACCACAAAGAGAGCUGCAACGGCCUGUACUACCAAGGCUC
UUGUUACAUCCUGCACAGCGACUAUAAGAGCUUCGAGGAUGCUAAGGCCAAUUGC
GCCGCCGAGAGCAGCACACUGCCUAACAAGUCCGACGUGCUGACCACCUGGCUGAU
CGACUACGUGGAAGAUACAUGGGGAAGCGACGGCAACCCCAUCACAAAAACCACAA
GCGAUUAUCAGGACAGCGACGUGUCUCAGGAGGUUAGAAAGUACUUUUGCACC
SEQ ID NO: 26 (the mRNA sequence of M1R peptide fragment) 
GCCGCCGCCUCUAUCCAGACCACAGUGAACACCCUGAGCGAGCGGAUCAGCUCCAA
GCUGGAGCAGGAGGCAAACGCAAGCGCCCAGACAAAGUGCGACAUCGAGAUCGGC
AAUUUCUACAUCAGACAGAACCACGGCUGCAAUAUCACCGUGAAGAAUAUGUGCU
CCGCCGACGCAGAUGCACAGCUGGAUGCCGUGCUGUCCGCCGCAACCGAGACAUAC
UCUGGCCUGACCCCUGAGCAGAAGGCCUAUGUGCCAGCCAUGUUCACAGCCGCCCU
GAACAUCCAGACCAGCGUGAAUACAGUGGUGCGGGACUUUGAGAACUACGUGAAG
CAGACCUGCAAUUCUAGCGCCGUGGUGGACAACAAGCUGAAGAUCCAGAAUGUGA
UCAUCGAUGAGUGUUAUGGAGCACCAGGCUCCCCUACCAACCUGGAGUUCAUCAAU
ACAGGCUCCUCUAAGGGCAACUGCGCCAUCAAGGCCCUGAUGCAGCUGACCACAAA
GGCCACCACACAGAUCGCACCUAGGCAGGUGGCAGGC
SEQ ID NO: 27(the mRNA sequence of B6R peptide fragment)

ACGUGCACGGUGCCCACGAUGAACAACGCGAAGUUGACGUCCACGGAAACCUCUUU
CAAUGAUAAACAAAAAGUCACGUUCACAUGUGACUCCGGAUACCACUCUUUGGAC
CCGAACGCAGUUUGUGAAACAGAUAAGUGGAAGUACGAGAAUCCGUGCAAGAAAA
UGUGCACGGUGUCGGAUUAUGUAUCAGAGUUGUACGACAAGCCGUUGUACGAGGU
UAAUAGUACCAUGACCUUGUCGUGCAACGGCGAAACCAAGUAUUUCCGUUGUGAA
GAGAAAAACGGGAACACGUCGUGGAACGACACGGUAACCUGCCCCAACGCGGAGU
GUCAACCUCUGCAGUUGGAGCACGGCUCUUGUCAGCCGGUGAAGGAAAAAUAUUC
CUUCGGCGAGUACAUGACUAUAAACUGUGAUGUGGGGUACGAGGUUAUAGGCGUA
UCCUACAUUAGCUGCACCGCUAAUAGUUGGAACGUCAUCCCCUCGUGCCAGCAGAA
AUGCGACAUUCCGUCUCUGUCAAAUGGCCUGAUCUCCGGGUCGACCUUCUCGAUAG
GUGGGGUUAUUCAUUUGUCCUGUAAGAGUGGGUUCACUCUUACAGGCUCCCCAUC
UUCGACCUGCAUAGAUGGGAAGUGGAACCCCAUCCUUCCCACUUGUGUCAGGUCGA
ACGAGGAAUUCGAUCCGGUAGAUGACGGCCCUGACGACGAGACGGAUCUGUCGAA
GCUGUCCAAAGACGUGGUUCAGUAUGAGCAGGAGAUAGAAUCUCUUGAGGCUACU
UACCAC

## Claims

1. A multi-immunogen chimeric or mixed poxvirus antigen, wherein the chimeric or mixed poxvirus antigen comprises:
an immunogen I: a monkeypox virus A35R protein or an antigenic fragment thereof, or an amino acid sequence having at least 90%, 92%, 95%, 96%, 97%, 98%, or 99% identity thereto and having the same or substantially the same immunogenicity therewith;
an immunogen II: a monkeypox virus M1R protein or an antigenic fragment thereof, or an amino acid sequence having at least 90%, 92%, 95%, 96%, 97%, 98%, or 99% identity thereto and having the same or substantially the same immunogenicity therewith; and
an immunogen III: a monkeypox virus B6R protein or an antigenic fragment thereof, or an amino acid sequence having at least 90%, 92%, 95%, 96%, 97%, 98%, or 99% identity thereto and having the same or substantially the same immunogenicity therewith.

2. The multi-immunogen chimeric or mixed poxvirus antigen according to claim 1, wherein the antigenic fragment of the A35R protein is an extracellular segment of the protein or a part thereof, preferably a peptide fragment having an amino acid sequence shown in SEQ ID NO: 1, or an amino acid sequence consisting of SEQ ID NO: 1 plus a fragment of 1 to 30 amino acids extending therefrom toward an N-terminus of the A35R protein, which is preferably an amino acid sequence shown in SEQ ID NO: 2;
and/or, the antigenic fragment of the M1R protein is an extracellular segment of the protein or a part thereof, preferably a peptide fragment having an amino acid sequence shown in SEQ ID NO: 3;
and/or, the antigenic fragment of the B6R protein is an extracellular segment of the protein or a part thereof, preferably a peptide fragment having an amino acid sequence shown in SEQ ID NO: 4.

3. The multi-immunogen chimeric or mixed poxvirus antigen according to claim 1 or 2, wherein the antigen is a multi-immunogen mixed antigen which is a mixture of the immunogens I, II, and III;
wherein preferably, the immunogen I has an amino acid sequence shown in SEQ ID NO: 1 or SEQ ID NO: 2, or an amino acid sequence that is derived from the amino acid sequence shown in SEQ ID NO: 1 or SEQ ID NO: 2 by substitution, deletion, or addition of one or several amino acids and has the same or substantially the same immunogenicity therewith;
and/or, the immunogen II has an amino acid sequence shown in SEQ ID NO: 3, or an amino acid sequence that is derived from the amino acid sequence shown in SEQ ID NO: 3 by substitution, deletion, or addition of one or several amino acids and has the same or substantially the same immunogenicity therewith;
and/or, the immunogen III has an amino acid sequence shown in SEQ ID NO: 4, or an amino acid sequence that is derived from the amino acid sequence shown in SEQ ID NO: 4 by substitution, deletion, or addition of one or several amino acids and has the same or substantially the same immunogenicity therewith, which is preferably an amino acid sequence shown in SEQ ID NO: 5; and
preferably, a mass ratio of the immunogens I, II, III in the mixture is 1:1:1.

4. The multi-immunogen chimeric or mixed poxvirus antigen according to claim 1 or 2, wherein the antigen is a multi-immunogen chimeric antigen which is a single chain composed of one or more immunogens I, II, and III attached in tandem.

5. The multi-immunogen chimeric or mixed poxvirus antigen according to claim 4, wherein the chimeric antigen comprises an amino acid sequence arranged in a form of A1-C₁-M-C₂-A2-C₃-B, wherein:
A1 represents a monkeypox virus A35R protein or an antigenic fragment I thereof, or an amino acid sequence having at least 90%, 92%, 95%, 96%, 97%, 98%, or 99% identity thereto and having the same or substantially the same immunogenicity therewith;
A2 represents the monkeypox virus A35R protein or an antigenic fragment II thereof, or an amino acid sequence having at least 90%, 92%, 95%, 96%, 97%, 98%, or 99% identity thereto and having the same or substantially the same immunogenicity therewith;
M represents a monkeypox virus M1R protein or an antigenic fragment thereof, or an amino acid sequence having at least 90%, 92%, 95%, 96%, 97%, 98%, or 99% identity thereto and having the same or substantially the same immunogenicity therewith,
B represents a monkeypox virus B6R protein or an antigenic fragment thereof, or an amino acid sequence having at least 90%, 92%, 95%, 96%, 97%, 98%, or 99% identity thereto and having the same or substantially the same immunogenicity therewith;
C₁, C₂, and C₃ are each independently none, or a linker sequence (GGGGS)m, wherein m is any integer between 1 and 10; and
wherein:
A1 is the same as or different from A2.

6. The multi-immunogen chimeric or mixed poxvirus antigen according to claim 5, wherein the A1 represents an amino acid sequence shown in SEQ ID NO: 1, or an amino acid sequence that is derived from the amino acid sequence shown in SEQ ID NO: 1 by substitution, deletion, or addition of one or several amino acids and has the same or substantially the same immunogenicity therewith;
and/or, the A2 represents an amino acid sequence shown in SEQ ID NO: 1, or an amino acid sequence consisting of SEQ ID NO: 1 plus a fragment of 1 to 30 amino acids extending therefrom toward an N-terminus of the A35R protein, which is preferably an amino acid sequence shown in SEQ ID NO: 2, or an amino acid sequence that is derived from any of the amino acid sequences by substitution, deletion, or addition of one or several amino acids and has the same or substantially the same immunogenicity therewith;
and/or, the M represents an amino acid sequence shown in SEQ ID NO: 3, or an amino acid sequence that is derived from the amino acid sequence shown in SEQ ID NO: 3 by substitution, deletion, or addition of one or several amino acids and has the same or substantially the same immunogenicity therewith;
and/or, the B represents an amino acid sequence shown in SEQ ID NO: 4, or an amino acid sequence that is derived from the amino acid sequence shown in SEQ ID NO: 4 by substitution, deletion, or addition of one or several amino acids and has the same or substantially the same immunogenicity therewith, which is preferably an amino acid sequence shown in SEQ ID NO: 5.

7. The multi-immunogen chimeric or mixed poxvirus antigen according to claim 6, wherein the A1 represents the amino acid sequence shown in SEQ ID NO: 1, the A2 represents the amino acid sequence shown in SEQ ID NO: 1 or SEQ ID NO: 2, the M represents the amino acid sequence shown in SEQ ID NO: 3, and the B represents the amino acid sequence shown in SEQ ID NO: 4 or SEQ ID NO: 5;
preferably, the A1 represents the amino acid sequence shown in SEQ ID NO: 1, the A2 represents the amino acid sequence shown in SEQ ID NO: 2, the M represents the amino acid sequence shown in SEQ ID NO: 3, and the B represents the amino acid sequence shown in SEQ ID NO: 4 or SEQ ID NO: 5;
preferably, the C₁, C₂, C₃ are all none; and
further preferably, the chimeric antigen comprises an amino acid sequence shown in SEQ ID NO: 6 or SEQ ID NO: 7.

8. The multi-immunogen chimeric or mixed poxvirus antigen according to claim 4, wherein the chimeric antigen comprises an amino acid sequence arranged in a form of A-C₁'-M-C₂'- B, wherein:
A represents a monkeypox virus A35R protein or an antigenic fragment thereof, or an amino acid sequence having at least 90%, 92%, 95%, 96%, 97%, 98%, or 99% identity thereto and having the same or substantially the same immunogenicity therewith;
M represents a monkeypox virus M1R protein or an antigenic fragment thereof, or an amino acid sequence having at least 90%, 92%, 95%, 96%, 97%, 98%, or 99% identity thereto and having the same or substantially the same immunogenicity therewith,
B represents a monkeypox virus B6R protein or an antigenic fragment thereof, or an amino acid sequence having at least 90%, 92%, 95%, 96%, 97%, 98%, or 99% identity thereto and having the same or substantially the same immunogenicity therewith;
C₁' and C₂' are each independently none, or a linker sequence (GGGGS)n, wherein n is any integer between 1 and 10.

9. The multi-immunogen chimeric or mixed poxvirus antigen according to claim 8, wherein the A represents an amino acid sequence shown in SEQ ID NO: 1, or an amino acid sequence that is derived from the amino acid sequence shown in SEQ ID NO: 1 by substitution, deletion, or addition of one or several amino acids and has the same or substantially the same immunogenicity therewith;
and/or, the M represents an amino acid sequence shown in SEQ ID NO: 3, or an amino acid sequence that is derived from the amino acid sequence shown in SEQ ID NO: 3 by substitution, deletion, or addition of one or several amino acids and has the same or substantially the same immunogenicity therewith;
and/or, the B represents an amino acid sequence shown in SEQ ID NO: 4, or an amino acid sequence that is derived from the amino acid sequence shown in SEQ ID NO: 4 by substitution, deletion, or addition of one or several amino acids and has the same or substantially the same immunogenicity therewith, which is preferably an amino acid sequence shown in SEQ ID NO: 5.

10. The multi-immunogen chimeric or mixed poxvirus antigen according to claim 9, wherein the A represents the amino acid sequence shown in SEQ ID NO: 1, the M represents the amino acid sequence shown in SEQ ID NO: 3, and the B represents the amino acid sequence shown in SEQ ID NO: 4 or SEQ ID NO: 5;
preferably, the C₁' and C₂' are both none; and
further preferably, the chimeric antigen comprises an amino acid sequence shown in SEQ ID NO: 8 or SEQ ID NO: 9.

11. The multi-immunogen chimeric or mixed poxvirus antigen according to any one of claims 4 to 10, wherein the chimeric antigen further comprises a signal peptide sequence at its N-terminus; and optionally, the signal peptide sequence is as shown in SEQ ID NO: 19.

12. A preparation method for the multi-immunogen chimeric poxvirus antigen of any one of claims 4 to 10, comprising the steps of: adding a Kozak sequence and a signal peptide coding sequence to a 5' terminus of a nucleotide sequence encoding the chimeric poxvirus antigen of any one of claims 4 to 10, adding a His-tag coding sequence and a stop codon to a 3' terminus thereof, performing cloning and expression, selecting correct recombinants, then transfecting the correct recombinants into an expression system cell for expression, collecting a cell culture supernatant, and isolating the chimeric antigen therefrom.

13. The preparation method according to claim 12, wherein the expression system cell is a mammalian cell, an insect cell, a yeast cell, or a bacterial cell;
optionally, the mammalian cell is an HEK293T cell, a 293F cell, or a CHO cell; further optionally, the 293F cell is an HEK293F cell, a Freestyle293F cell, or an Expi293F cell;
optionally, the insect cell is an sf9 cell, an Hi5 cell, an sf21 cell, or an S2 cell;
optionally, the yeast cell is a *Pichia pastoris* cell or a yeast cell modified therefrom; and
optionally, the bacterial cell is an *Escherichia coli* cell.

14. A polynucleotide encoding the multi-immunogen chimeric or mixed poxvirus antigen of any one of claims 1 to 11.

15. The polynucleotide according to claim 14, wherein the polynucleotide is DNA or mRNA;
when encoding the multi-immunogen chimeric poxvirus antigen, preferably, the polynucleotide is a DNA molecule comprising or consisting of a DNA sequence shown in one of SEQ ID NOs: 10, 11, 12, or 13; alternatively, the polynucleotide is an mRNA molecule comprising or consisting of an mRNA sequence shown in one of SEQ ID NOs: 14, 15, 16, or 17;
when encoding the multi-immunogen mixed poxvirus antigen, preferably, the polynucleotide is a polynucleotide group comprising or consisting of: a DNA molecule comprising a DNA sequence shown in SEQ ID NO: 22, a DNA molecule comprising a DNA sequence shown in SEQ ID NO: 23, and a DNA molecule comprising a DNA sequence shown in SEQ ID NO: 24; alternatively, the polynucleotide group comprises or consists of: an mRNA molecule comprising an mRNA sequence shown in SEQ ID NO: 25, an mRNA molecule comprising an mRNA sequence shown in SEQ ID NO: 26, and an mRNA molecule comprising an mRNA sequence shown in SEQ ID NO: 27.

16. A nucleic acid construct comprising the polynucleotide of claim 14 or 15, and optionally at least one expression regulatory element operably linked to the polynucleotide.

17. An expression vector comprising the nucleic acid construct of claim 16.

18. A host cell in which the polynucleotide of claim 14 or 15, the nucleic acid construct of claim 16, or the expression vector of claim 17 is transformed or transfected.

19. Use of the multi-immunogen chimeric or mixed poxvirus antigen of any one of claims 1 to 11, the polynucleotide of claim 14 or 15, the nucleic acid construct of claim 16, the expression vector of claim 17, or the host cell of claim 18 in the preparation of a medicament for the prevention and/or treatment of poxvirus infections;
preferably, the poxvirus is selected from the group consisting of monkeypox virus, smallpox virus, cowpox virus, and/or vaccinia virus;
optionally, the medicament is a vaccine, preferably an mRNA vaccine;
optionally, the vaccine is in a form of a nasal spray, an oral formulation, a suppository, or a parenteral formulation;
preferably, the nasal spray is selected from the group consisting of an aerosol, a spray, and a dry powder inhaler;
preferably, the oral formulation is selected from the group consisting of a tablet, a powder, a pill, granules, a soft/hard capsule, a film-coated agent, and an ointment; further preferably, the tablet is a sublingual tablet; further preferably, the granules are fine granules; further preferably, the powder is a pulvis; further preferably, the pellet is a pilule;
preferably, the parenteral formulation is a transdermal formulation, an ointment, a plaster, a topical liquid, or an injectable formulation; and further preferably, the injectable formulation is a formulation for infusion.

20. A vaccine or immunogenic composition comprising the multi-immunogen chimeric or mixed poxvirus antigen of any one of claims 1 to 11, the polynucleotide of claim 14 or 15, the nucleic acid construct of claim 16, the expression vector of claim 17, or the host cell of claim 18, and a physiologically acceptable vehicle, adjuvant, excipient, carrier, and/or diluent.

21. The vaccine or immunogenic composition according to claim 20, which is a recombinant protein vaccine against a poxvirus and comprises the multi-immunogen chimeric or mixed poxvirus antigen of any one of claims 1 to 11 and an adjuvant; and
optionally, the adjuvant is one or more selected from the group consisting of an aluminum adjuvant, an MF59 adjuvant, and an MF59-like adjuvant.

22. The vaccine or immunogenic composition according to claim 20, which is a DNA vaccine against a poxvirus, comprising:
(1) a eukaryotic expression vector; and
(2) a DNA sequence constructed into the eukaryotic expression vector and encoding the multi-immunogen chimeric or mixed poxvirus antigen of any one of claims 1 to 11;
preferably, when encoding the multi-immunogen chimeric poxvirus antigen, the DNA sequence constructed into the eukaryotic expression vector is a DNA sequence shown in one of SEQ ID NOs: 10, 11, 12 or 13;
preferably, when encoding the multi-immunogen mixed poxvirus antigen, the DNA vaccine comprises three eukaryotic expression constructs, with the DNA sequence in each of the eukaryotic expression constructs being shown in SEQ ID NOs: 22, 23, 24, respectively; and optionally, the eukaryotic expression vector is selected from the group consisting of pGX0001, pVAX1, pCAGGS, and pcDNA series vectors.

23. The vaccine or immunogenic composition according to claim 20, which is an mRNA vaccine of a poxvirus, comprising:
(I) an mRNA molecule encoding the multi-immunogen chimeric or mixed poxvirus antigen of any one of claims 1 to 11; and
(II) lipid nanoparticles;
preferably, when encoding the multi-immunogen chimeric poxvirus antigen, the mRNA molecule has an mRNA sequence shown in one of SEQ ID NOs: 14, 15, 16, or 17;
preferably, when encoding the multi-immunogen mixed poxvirus antigen, the mRNA vaccine comprises three mRNA molecules encapsulated within lipid nanoparticles having mRNA sequences shown in SEQ ID NOs: 25, 26, 27, respectively.

24. The vaccine or immunogenic composition according to claim 20, which is a poxvirus-viral vector vaccine comprising:
(1) a viral backbone vector; and
(2) a DNA sequence constructed into the viral backbone vector and encoding the multi-immunogen chimeric or mixed poxvirus antigen of any one of claims 1-11;
preferably, when encoding the multi-immunogen chimeric poxvirus antigen, the DNA sequence constructed into the viral backbone vector is a DNA sequence shown in one of SEQ ID NOs: 10, 11, 12, or 13;
preferably, when encoding the multi-immunogen mixed poxvirus antigen, the poxvirus-viral vector vaccine comprises three virus vector constructs, with the DNA sequence in each of the viral vector constructs being shown in SEQ ID NOs: 22, 23, 24, respectively; and
optionally, the viral backbone vector is one or more selected from the following viral vectors: an adenovirus vector, a poxvirus vector, an influenza virus vector, and an adeno-associated virus vector.

25. The vaccine or immunogenic composition according to any one of claims 20 to 24, wherein the vaccine or immunogenic composition is in a form of a nasal spray, an oral formulation, a suppository, or a parenteral formulation;
preferably, the nasal spray is selected from the group consisting of an aerosol, a spray, and a dry powder inhaler;
preferably, the oral formulation is selected from the group consisting of a tablet, a powder, a pill, granules, a soft/hard capsule, a film-coated agent, and an ointment;
further preferably, the tablet is a sublingual tablet;
further preferably, the granules are fine granules;
further preferably, the powder is a pulvis;
further preferably, the pill is a pilule;
preferably, the parenteral formulation is a transdermal formulation, an ointment, a plaster, a topical liquid, or an injectable formulation; and further preferably, the injectable formulation is a formulation for infusion.
